# EUROPEAN PATENT SPECIFICATION

(11) **EP 4 526 660 B1**
(45) Date of publication and mention of the grant of the patent: **04.11.2026**
(21) Application number: 23728275.1
(22) Date of filing: 16.05.2023
(51) Int. Cl.: G01N 21/552, G01N 21/75, G01N 21/25

(54) **LOCALISED SURFACE PLASMONIC SENSING**
MESSUNG MITTELS LOKALISIERTER OBERFLÄCHENPLASMONENRESONANZ
DÉTECTION À RÉSONANCE DE PLASMONS DE SURFACE LOCALISÉS

(30) Priority: 16.05.2022 GB 202207147
(43) Date of publication of application: 26.03.2025
(60) Divisional application: 26207783.7
(73) Proprietor: The University Court Of The University Of Glasgow, Glasgow, Strathclyde G12 8QQ (GB)
(72) Inventor: CLARK, Alasdair, Glasgow Strathclyde G12 8QQ (GB); PEVELER, William, Glasgow Strathclyde G12 8QQ (GB); SPERLING, Justin, Glasgow Strathclyde G12 8QQ (GB); CHRISTIE, Iain Edgar, Glasgow G51 4RN (GB)
(74) Representative: Mewburn Ellis LLP
(86) International application number: PCT/EP2023/063146
(87) International publication number: WO 2023/222709

(56) References cited:
- WO-A1-2021/156346
- ROSMAN CHRISTINA ET AL: "Multiplexed Plasmon Sensor for Rapid Label-Free Analyte Detection", NANO LETTERS, vol. 13, no. 7, 26 June 2013 (2013-06-26), US, pages 3243 - 3247, XP093065776, ISSN: 1530-6984, DOI: 10.1021/nl401354f
- JI JIALIN ET AL: "Thermal annealing induced tunable localized surface plasmon resonance of Au/Ag bimetallic thin film", CHEMICAL PHYSICS, NORTH-HOLLAND, NL, vol. 541, 3 November 2020 (2020-11-03), XP086380001, ISSN: 0301-0104, [retrieved on 20201103], DOI: 10.1016/J.CHEMPHYS.2020.111034
- MACIAS GERARD ET AL: "Whisky tasting using a bimetallic nanoplasmonic tongue", NANOSCALE, vol. 11, no. 32, 1 January 2019 (2019-01-01), United Kingdom, pages 15216 - 15223, XP093068733, ISSN: 2040-3364, DOI: 10.1039/C9NR04583J

## Description

### Field of the Invention

The present invention relates to the sensing of analytes using a localised surface plasmonic sensing device, and relates to a localised surface plasmonic sensing device . The invention is of particular, although not necessarily exclusive, interest in the characterisation of mixtures of analytes.

### Background

Various known electronic sensing devices are designed to resemble and enhance the biological senses. Photodetectors, pressure and temperature sensors, and microphones can be related to the biological counterparts of vision, touch, and hearing, respectively. However, there are still two senses that are extremely challenging to replicate: smell and taste. These senses are highly capable for detecting individual components in complex chemical mixtures or differentiating and grouping different mixtures. Chromatography is the current gold-standard for detection, identification, and classification of chemical components from complex gas and liquid mixtures. However, the nature of chromatographic identification techniques (such as liquid chromatography mass spectrometry) requires specialized laboratory equipment for the separation and analysis of a sample's chemical components. This results in costly, time-consuming, and often low throughput processes that are unsuitable for applications where real-time monitoring (or near-real-time monitoring) and/or portability are desirable (air and liquid sampling in the security, food, or drug sectors, for example). An example of the use of a gas chromatography system is shown in Ref A1. An example of the use of a liquid chromatography system is shown in Ref A2.

In response to these issues artificial 'tongues' and 'noses' consisting of multiple, cross-reacting sensing elements have been developed (Refs. A3-A8). Compared to the specialized laboratory equipment mentioned above (Refs. A1 and A2), these devices are portable, highly sensitive (Ref. A9), do not require component isolation, and can be fabricated relatively cheaply (Ref. A10, Ref. A11).

Previous work in this technical field arising from the inventors' research group has been published as WO 2021/156346 (Ref. A12) and in Ref. A13. These disclosures set out the use of interspersed functionalised gold and aluminium nano-arrays as plasmonic arrays. This bimetallic device produced two distinct resonance peaks for each sensing region. Using these plasmonic arrays, the authors were able to demonstrate differentiation between off-the-shelf whiskies by means of linear discriminant analysis (LDA).

An example of a sensor device based on gold nanostructures is disclosed in US 2011/0164252 A1 (Ref. A14). The gold nanostructures are arranged as islands on a light-transmitting substrate. The gold nanostructures have surface functionalisation in order to bind to an analyte present in an analysis liquid that is brought into contact with the gold nanostructures. The sensor device is illuminated with polarised light and detected by a spectroscopic optical system. This illumination and detection is carried out before and after the analysis liquid is brought into contact with the gold nanostructures, in order to provide a reference spectrum for comparison.

The publication: "Multiplexed Plasmon Sensor for Rapid Label-Free Analyte Detection", Rosman Christina et al: Nano Letters, vol. 13, no. 7, pages 3243-3247, 26 June 2013, ISSN:1530-6984, DOI: 10.1021/nl401354f discloses a multiplexed sensor with randomly deposited aptamer functionalized gold nanorods.

### Summary of the Invention

The inventors consider that the work disclosed in Refs. A12 and A13 provides an interesting proof-of-concept for the use of arrays of localised surface plasmon resonance island structures in the differentiation of different liquids such as beverages. The intention behind the use of gold and aluminium island structures together was to allow facile differentiated surface functionalisation of the gold island structures compared with the aluminium island structures. However, the inventors have found that the use of aluminium island structures at the nanoscale may have drawbacks. In practical use, aluminium nanostructures oxidise relatively quickly, rendering them ineffective as localised surface plasmon resonance island structures and/or affecting their surface functionalisation.

The inventors further consider that additional improvements over the work disclosed in Refs. A12 and A13 are possible to provide a more practical and useful sensing device.

The present invention has been devised in light of the above considerations.

The current invention provides a localised surface plasmonic sensing device according to appended claim 1.

In some preferred embodiments, each localised surface plasmon resonance island structure comprises gold, for example as a gold layer formed on a titanium layer itself formed on the substrate (which may be, for example, glass). As gold does not easily adhere to glass, where a glass substrate is used a titanium interlayer is preferred.

Suitably, each localised surface plasmon resonance island structure has the same shape as the others. In some preferred embodiments, each localised surface plasmon resonance island structure comprises at least two distinct pieces, the pieces not being in contact with one another and being separated by a distance which is at least 5 nm and not more than 150 nm.

For example, it may be preferred that each localised surface plasmon resonance island structure comprises at least three distinct pieces, the pieces not being in contact with one another and being separated by a distance which is at least 5 nm and not more than 150 nm.

In these types of embodiment, each piece of a given localised surface plasmon resonance island structure may an arcuate shape or a circular shape.

In particular designs, either: (i) each piece of a given localised surface plasmon resonance island structure has a shape corresponding to a portion of a ring; the ring being formed by the pieces of the localised surface plasmon resonance island structure and the spaces between those pieces, the spaces giving a piece-to-piece size of at least 5 nm and not more than 150 nm; or (ii) each piece of a given localised surface resonance island structure has a circular shape, the circular pieces being separated by a space giving a piece-to-piece size of at least 5 nm and not more than 150 nm.

In the present invention, it may be preferred that each localised surface plasmon resonance island structure has been subjected to an annealing process.

In the present invention, each surface functionalisation is preferably derived from a sulfur containing compound; optionally, wherein each surface functionalisation is derived from a functionalising compound that is a thiol or a disulfide.

In some embodiments each surface functionalisation is derived from a functionalising compound that is a thiol or a disulfide and that contains at least one of a carboxylic acid group COOH, an alcohol group OH, a ketone group CO, an amine group NH₂, an amide group, an aliphatic group, an aromatic group, a nitro group NO₂ or a boronic acid group B(OH)₂.

In some embodiments the surface functionalisations on the substrate are selected such that, amongst their number, at least an alcohol group OH; an amine group NH₂; an aliphatic group; an aromatic group; and a halogen group (F, Cl or Br) are present.

In some embodiments the surface functionalisations on the substrate include at least a surface functionalisation derived from 1-Dodecanethiol, a surface functionalisation derived from 6-mercapto-1-hexanol, a surface functionalisation derived from 3-amino-5-mercapto-1,2,4-triazole, and a surface functionalisation derived from 3,4-dichlorothiophenol.

The present localised surface plasmonic sensing devices may in some embodiments further comprise: a fifth array of localised surface plasmon resonance island structures on the substrate; a sixth array of localised surface plasmon resonance island structures on the substrate; a seventh array of localised surface plasmon resonance island structures on the substrate; wherein: the first, second, third, fourth, fifth, sixth and seventh arrays are located to be spaced apart and isolated from each other on the substrate; the localised surface plasmon resonance island structures of the first, second, third, fourth, fifth, sixth and seventh array respectively have first, second, third, fourth, fifth, sixth and seventh surface functionalisations for selective interaction with respective analytes; the first, second, third, fourth, fifth, sixth and seventh surface functionalisations are different to each other and, other than the different surface functionalisation, the localised surface plasmon resonance island structures of the first, second, third, fourth, fifth, sixth and seventh array have the same composition as each other.

In such embodiment, the surface functionalisations on the substrate may suitably include at least a surface functionalisation derived from 1H,1H,2H,2H-perfluorodecanethiol, a surface functionalisation derived from 1-Dodecanethiol, a surface functionalisation derived from 4-mercaptobenzoic acid, a surface functionalisation derived from 6-mercapto-1-hexanol, a surface functionalisation derived from 3-amino-5-mercapto-1,2,4-triazole, a surface functionalisation derived from 4-nitrothiophenol and a surface functionalisation derived from 3,4-dichlorothiophenol.

A second aspect of the invention relates to a method for manufacturing a localised surface plasmonic sensing device, the method comprising the steps: providing a substrate; forming a first array of localised surface plasmon resonance island structures on the substrate; forming a second array of localised surface plasmon resonance island structures on the substrate; forming a third array of localised surface plasmon resonance island structures on the substrate; forming a fourth array of localised surface plasmon resonance island structures on the substrate, wherein the first, second, third and fourth arrays are substantially identical; wherein the first, second, third and fourth arrays are located to be spaced apart and isolated from each other on the substrate, the method further comprising the step: modifying the localised surface plasmon resonance island structures of the first, second, third and fourth array respectively to provide first, second, third and fourth surface functionalisation for selective interaction with respective analytes, wherein the first, second, third and fourth surface functionalisation are different to each other.

In some embodiments, between the step of forming a given array and the step of modifying the localised surface plasmon resonance island structures of that array, the method further comprises an annealing step of heating the array in an inert atmosphere at a temperature of 300 to 700°C for 300 to 1200 seconds.

In some embodiments, the step of modifying the localised surface plasmon resonance island structures of the first, second, third and fourth arrays is done by applying solutions of respective functionalising compounds dropwise to the arrays, followed by washing of the arrays.

A third aspect of the present invention provides a method of analysing a fluid comprising a mixture of two or more analytes, including the step of providing a localised surface plasmonic sensing device as described herein, the method further comprising the steps: contacting the arrays with said fluid comprising a mixture of two or more analytes and thereby allowing the analytes selectively to interact with the surface functionalisation available on the arrays of localised surface plasmon resonance island structures; illuminating the arrays with electromagnetic radiation to cause localised surface plasmon resonance; and receiving reflected or transmitted electromagnetic radiation from the arrays and detecting said localised surface plasmon resonance to analyse one or more characteristics of said analytes.

A fourth aspect of the present invention provides a method of analysing a fluid to detect the presence and/or concentration of at least one analyte, including the step of providing a localised surface plasmonic sensing device comprising: a substrate; a first array of localised surface plasmon resonance island structures on the substrate; a second array of localised surface plasmon resonance island structures on the substrate; wherein: the first and second arrays are located to be spaced apart and isolated from each other on the substrate; the localised surface plasmon resonance island structures of the first and second array respectively have first and second surface functionalisation with different interaction with said at least one analyte; the first and second surface functionalisation are different to each other and, other than the different surface functionalisation, the localised surface plasmon resonance island structures of the first and second array have the same composition as each other, the method further comprising the steps: contacting the arrays with said fluid and thereby allowing the analyte selectively to interact with the surface functionalisation available on the arrays of localised surface plasmon resonance island structures; illuminating the arrays with electromagnetic radiation to cause localised surface plasmon resonance; and receiving reflected or transmitted electromagnetic radiation from the arrays; measuring absorption spectra for the reflected or transmitted electromagnetic radiation from the respective arrays; determining from each absorption spectrum at least two or more spectral characteristics of: a minimum wavelength corresponding to an absorption peak; a full width at half maximum characteristic of an absorption peak; a lower wavelength corresponding to one flank of said absorption peak at said half maximum; an upper wavelength corresponding to the other flank of said absorption peak at said half maximum; and a ratio of said lower wavelength to said upper wavelength; and using said spectral characteristics to determine the presence and/or concentration of said at least one analyte in the fluid.

In some embodiments the method of analysing a fluid to detect the presence and/or concentration of at least one analyte comprises providing a third array of localised surface plasmon resonance island structures on the substrate; a fourth array of localised surface plasmon resonance island structures on the substrate; a fifth array of localised surface plasmon resonance island structures on the substrate; a sixth array of localised surface plasmon resonance island structures on the substrate; a seventh array of localised surface plasmon resonance island structures on the substrate; wherein: the first, second, third, fourth, fifth, sixth and seventh arrays are located to be spaced apart and isolated from each other on the substrate; the localised surface plasmon resonance island structures of the first, second, third, fourth, fifth, sixth and seventh array respectively have first, second, third, fourth, fifth, sixth and seventh surface functionalisations for different interaction with said at least one analyte; the first, second, third, fourth, fifth, sixth and seventh surface functionalisations are different to each other and, other than the different surface functionalisation, the localised surface plasmon resonance island structures of the first, second, third, fourth, fifth, sixth and seventh array have the same composition as each other; and wherein the different interaction with said at least one analyte by first, second, third, fourth, fifth, sixth and seventh surface functionalisations is other than by specific binding of the at least one analyte.

In such an embodiment the surface functionalisations on the substrate may include at least a surface functionalisation derived from 1H,1H,2H,2H-perfluorodecanethiol, a surface functionalisation derived from 1-Dodecanethiol, a surface functionalisation derived from 4-mercaptobenzoic acid, a surface functionalisation derived from 6-mercapto-1-hexanol, a surface functionalisation derived from 3-amino-5-mercapto-1,2,4-triazole, a surface functionalisation derived from 4-nitrothiophenol and a surface functionalisation derived from 3,4-dichlorothiophenol.

In some embodiments, it may be that the spectral characteristics of the method of analysing a fluid to detect the presence and/or concentration of at least one analyte are used to perform multivariate analysis to determine the presence and/or concentration of said at least one analyte in the fluid. It may be that the multivariate analysis is discriminant analysis.

A fluid being analysed by the method of analysing a fluid to detect the presence and/or concentration of at least one analyte may be a beverage, and optionally it may be an alcoholic beverage. It may be that the beverage is a beer or a spirit.

In a fifth aspect, the present invention relates to a localised surface plasmonic sensing device comprising: a substrate; a first array of localised surface plasmon resonance island structures on the substrate; a second array of localised surface plasmon resonance island structures on the substrate; a third array of localised surface plasmon resonance island structures on the substrate; a fourth array of localised surface plasmon resonance island structures on the substrate, wherein: the first, second, third and fourth arrays are located to be spaced apart and isolated from each other on the substrate; the localised surface plasmon resonance island structures of the first, second, third and fourth array respectively have first, second, third and fourth surface functionalisations for selective interaction with respective analytes; the first, second, third and fourth surface functionalisations are different to each other and, other than the different surface functionalisation, the localised surface plasmon resonance island structures of the first, second, third and fourth array have the same composition as each other; and wherein the surface functionalisations on the substrate include at least: a surface functionalisation derived from 1-Dodecanethiol, a surface functionalisation derived from 6-mercapto-1-hexanol, a surface functionalisation derived from 3-amino-5-mercapto-1,2,4-triazole, and a surface functionalisation derived from 3,4-dichlorothiophenol.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Fig. 1** is a schematic plan view of a surface plasmonic sensing device according to an embodiment of the invention;
**Fig. 2** is a schematic view of a surface resonance plasmonic sensing apparatus according to an embodiment of the invention;
**Fig. 3** is a schematic illustration of two possible nanostructure designs, with square (A) and split ring (B) shapes;
**Fig. 4** is an SEM image of exemplary deposited split ring nanostructures;
**Fig. 5** is an SEM image of exemplary deposited trimer nanostructures;
**Fig. 6** is SEM images of exemplary deposited square (left) and split ring (right) nanostructures with dimensions added;
**Fig. 7** is spectrophotometer spectra for a series of sensors and test solutions;
**Fig. 8** is a graph showing shifts of peak wavelength in spectrophotometric spectra for a series of sensor arrays and test solutions;
**Fig. 9** is a plot of peak shift from water against refractive index for the data in **Fig. 7****;**
**Fig. 10** is SEM images of (A) square design nanostructures before annealing and (B) the same structures after annealing;
**Fig. 11** is SEM images of (A) split ring design nanostructures before annealing and (B) the same structures after annealing;
**Fig. 12** is spectrophotometer spectra for square (A) and split ring (B) nanostructure designs before and after annealing;
**Fig. 13** is a plot of the first two canonical axes from LDA analysis of certain test solutions using a surface plasmonic sensing device according to an embodiment of the invention;
**Fig. 14** is a plot of the first three canonical axes from LDA analysis of certain test solutions using a surface plasmonic sensing device according to an embodiment of the invention;
**Fig. 15** is a plot of the nine canonical axes from LDA analysis of certain test solutions using a surface plasmonic sensing device according to an embodiment of the invention;
**Fig. 16** illustrates schematically the design of the photolithography masks and alignment markers used in a method of manufacturing a surface plasmonic sensing device according to an embodiment of the invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

Certain terms are used herein to refer to the arrangement of surface design features of the present devices. For the avoidance of doubt, significant ones of those terms will be explained here.

Nanostructure: also referred to as a localised surface plasmon resonance island structure. A single structure which can be functionalised. For example, a single split ring nanostructure or a single square nanostructure.

Nanoarray: a pattern made up of nanostructures; for example, a 2x2 lattice of square nanostructures would include 4 nanostructures and would be referred to as a single nanoarray. For consistency of wording we note here that a nanoarray may be a single nanostructure (that is, a nanoarray can be a single nanostructure or can include two or more nanostructures).

Nanophotonic region: a single, usually individually addressable site/location/'well' which includes at least one (indeed, suitably, exactly one) nanoarray of nanostructures.

Array: a pattern made up of nanophotonic regions; including a single nanophotonic region (that is, an array can be a single nanophotonic region or can include two or more nanophotonic regions).

From this discussion it will be apparent that, at its simplest, an "array" may include only a single localised surface plasmon resonance island structure; however, in general it will include more than one. For example, the array may comprise multiple nanophotonic regions (each having only a single nanoarray or even only a single nanostructure); or the array may comprise a single nanophotonic region which itself has multiple nanostructures within it.

The preferred embodiments of the invention utilise metallic nanostructures. These are considered to be of particular use in optical tongue devices thanks to their chemical stability, the sensitivity of their plasmonic resonance to environmental changes, and their ease of chemical-functionalization.

The embodiments described here provide a sensing device which uses the phenomenon of localised surface plasmon resonance. It is referred to as a surface plasmonic sensing device and also as an "optical tongue" device. The device is preferably reusable. The device preferably comprises gold nano-arrays; that is, gold is a preferred metal for the localised surface plasmon resonance island structures.

### Device Design and Usage

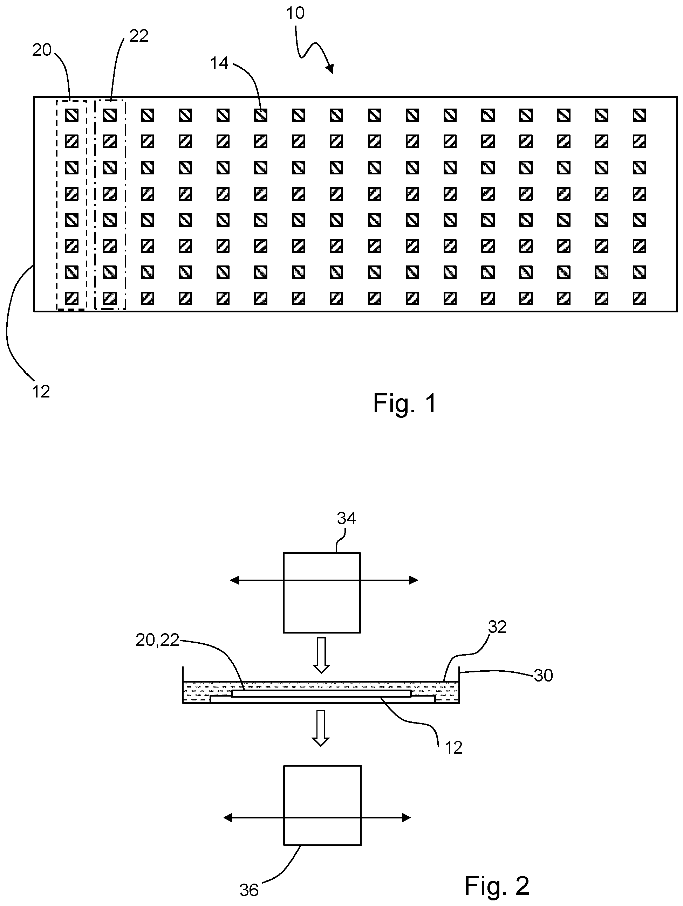

**Fig. 1** shows a schematic plan view of a surface plasmonic sensing device 10 according to an embodiment of the invention. The device comprises a substrate 12, typically based on glass. The device has a first array 20 of nanophotonic regions 14 on the substrate and a second array 22 of nanophotonic regions 14 on the substrate.

These arrays are preferably arranged in a columnwise fashion; that is, a single array is a column of multiple linearly aligned nanophotonic regions. Each column is separated from each other column. In this embodiment the nanophotonic regions in each column are also aligned in rows, to form an overall lattice/matrix arrangement.

While the first and second arrays are identified with reference numerals 20 and 22, it can be seen that there are, in this illustrated embodiment, sixteen such arrays, each representing a column of (in this example) eight nanophotonic regions. Of course, it will be recognised that the number of arrays (i.e. number of different functionalisations to be used in the device) and the number of nanophotonic regions per array can be varied depending on the desired performance of the device. For example, there is no limit on the number of nanophotonic regions an array might have; it might have, for example, 1-100, for example at least 2, at least 4, at least 8, at least 10, at least 16, at least 32, or at least 64. The number of arrays in the present invention is, at its broadest, at least 4; it may be, for example, at least 5, at least 6, at least 7, at least 8, at least 9, at least 10, at least 11, at least 12, at least 13, at least 14, at least 15 or at least 16. The specific functionalisations chosen may affect the desired (or required) number of arrays.

In this embodiment, each localised surface plasmon resonance island structure is a metallic nanostructure, having a square shape in plan view. Also in this embodiment, each array is ultimately formed of Au nanostructures (arranged as nanoarrays within the nanophotonic regions). The localised surface plasmon resonance island structures of the arrays respectively have first, second, third etc. surface functionalisations for selective interaction with respective analytes. Each surface functionalisation is preferably different to the other surface functionalisations.

**Fig. 2** shows a schematic view of a surface plasmonic sensing apparatus according to an embodiment of the invention. The surface plasmonic sensing device 12 is placed in a receptacle 30 that constitutes a fluid contacting arrangement to allow contacting of the arrays (schematically shown) with fluid 32 comprising a mixture of two or more analytes. The analytes thereby selectively interact with the surface functionalisations available on the localised surface plasmon resonance island structures.

In particular, in the present invention it is believed that functionalisations can act to segregate the fluid under analysis at the surface. Each given functionalisation will, by virtue of its particular chemistry, repel certain components of the fluid while not repelling (or even attracting) others. Accordingly the fluid is separated at a molecular level, to some degree, by the functionalisation. Only certain molecules are able to enter into the very small sensing volume around the nanostructure and hence influence the LSPR frequency.

The inventors believe that this mode of action is different from previously known sensors which specifically (chemically) bind target analytes to the surface for sensing. For example, the functionalisations of such surfaces may be specifically designed or engineered to bind a target analyte whose content in a given sample is to be investigated. The present functionalisations are generally not so designed or targeted based on an intended specific binding to a specific analyte. Examples of this specific sensing might include antibody-analyte binding or nucleic acid base-pairing. Accordingly the reusability and flexibility (i.e. ability to sense multiple different molecules) of such previous sensors is reduced, and sample analysis is only possible if the target is previously known and a suitable binding moiety can be generated. The present invention, using a different concept, where multiple selective but not specific interactions are generated across several sensing elements, does not necessarily suffer from those drawbacks. "Selective interaction" as used herein is understood to exclude pre-organised multivalent binding interactions. It will be apparent that functionalisations which do in fact bind certain molecules can still be useful in the present invention. However the present functionalisations are generally not chosen with specificity for any particular target analyte.

It may be that the functionalisations of the present invention are incapable of taking part in specific interactions. It may be that the functionalisations are not part of a cognate pair. It may be that the functionalisations of the present invention are not proteins, nucleic acids, or entities that are complementary in a specific way to a protein.

Because of the broad spectrum applicability of the present functionalisations (rather than being targeted at specific analytes), the present sensors can be used for a wide variety of samples including a wide variety of analytes. The present methods of analysing a fluid may utilise a localised surface plasmonic sensing device in which the functionalisation(s) are not chosen with regard to the fluid to be analysed; that is, not chosen to specifically bind particular analytes in the fluid.

Illumination source 34 (typically a broadband illumination source) is positionable selectively to illuminate the arrays. The receptacle 30 is formed of light-transmissive material, as is the substrate 12 of the device, and therefore detector 36 is located to receive transmitted light from the device to detect said localised surface plasmon resonance to analyse one or more characteristics of said analytes. Detector 36 is also positionable selectively to interrogate the arrays 20, 22 and so on.

### Nanostructure Design

Each localised surface plasmon resonance island structure (nanostructure) has a 'design', its general shape and size. This can be selected as appropriate when the nanostructures are being formed.

Preferably, for ease of manufacture and consistency/comparability of results, each nanostructure in given device has the same design, that is, the same general size and shape. Suitable sizes and shapes are known in the art.

For example, each nanostructure may be substantially circular, annular, square, rectangular, triangular or otherwise polygonal. Substantially square nanostructures are used in certain embodiments of the invention. For example, square nanostructures having a side length of 80 to 150 nm, preferably 100 to 130 nm are suitable.

The present inventors have, however, also employed nanostructure designs which can be utilised to achieved improved device performance, in particular in terms of sensitivity.

The inventors have found in particular that splitting a given nanostructure into multiple (for example, two or more, preferably three or more) distinct pieces or segments, closely spaced, can enhance the performance.

The inventors have found that, by careful design of the split nanostructure, a coupling of the resonance in one segment of the split nanostructure to the resonance in the neighbouring segment can occur.

The resonance is comprised of the free-electrons in the metal being resonantly 'driven' by the photons of the incident light (the resonance being set up when the photons and the electrons have matched oscillation frequencies).

When two discrete segments are positioned near to each other (each segment of the split nanostructure effectively being considered as a discrete structure in its own right) there can be coupling between the respective resonances in the segments. In other words, the oscillating electron clouds of each segment join together. This shifts the resonance frequency, and has been found to give a significant enhancement of the electric field inside the gap(s) between the segments. This drastically improves sensitivity of the sensor.

For example, a particularly preferred design identified by the inventors is of a 'split ring'. In this design, a substantially annular (i.e. ring shaped) nanostructure is deposited by split into three, suitably substantially equal, segments. Each segment is substantially arcuate, corresponding to a portion of the underlying ring shape. The segments are separated by spaces, those spaces also corresponding to portions of the underlying ring shape. Spaces at, broadly, 0°, 120°, and 240° can separate the ring into three substantially equal segments.

The spaces may be sized appropriately. They may have a size of for example 5 nm to 150 nm, preferably 5 nm to 40 nm, suitably 7 nm to 20 nm.

These sizes are particularly suitable when the 'ring' upon which the split ring design is based has a diameter of 150 to 300 nm, for example 170 to 250 nm or 200 to 220 nm.

The central (empty) core of the ring may suitably have a diameter of 100 to 130 nm. The radial thickness/width of each of the arcuate pieces of the split ring design may suitably be 10 to 100 nm, more suitably 20 to 75 nm, and yet more suitably 35 to 60 nm.

Another design the inventors have employed is a nanostructure made up of three distinct substantially circular or disc-shaped pieces, the three themselves being arranged such that their centres substantially form a triangle. This 'trimer' design means there are spaces between the three pieces. Suitably the spaces are each at least 5 nm and at most 150 nm

### Nanoarray Design

Each nanoarray comprises at least one nanostructure as set out above. Suitably each nanoarray comprises more than one nanostructure; the nanostructures then forming a pattern in the nanoarray.

Such a pattern may consist of a number of nanostructures arranges in a grid or lattice, with lines of nanostructures existing in orthogonal 'X' and 'Y' directions. Between adjacent nanostructures in the X and Y directions can be defined X and Y periods, corresponding to the centre-to-centre spacing between adjacent nanostructures in the X and Y directions.

The X period may suitably be in the range from 200 nm to 800 nm, more preferably 250 nm to 450 nm.

The Y period may suitably be in the range from 200 nm to 800 nm, more preferably 250 nm to 450 nm.

In some embodiments the X and Y periods are the same; in other embodiments the X and Y periods are different.

Particularly, where a substantially square nanostructure design is used the X and Y periods may preferably both be in the range from 250 nm to 350 nm, more preferably being about 300 nm (±5 nm). Where the above mentioned 'split ring' nanostructure design is used the X and Y periods may preferably both be in the range from 350 to 450 nm, more preferably being about 400 nm (±5 nm).

Looked at differently, the spacing of nanostructures within a nanoarray can be defined in terms of their edge-to-edge distance, that is, the shortest distance between any part of a given nanostructure and any part of the adjacent nanostructure in the X or Y direction. These gaps can be defined as X and Y spacings.

The X spacing may suitably be in the range from 100 nm to 500 nm, more preferably 150 nm to 250 nm.

The Y spacing may suitably be in the range from 100 nm to 500 nm, more preferably 150 nm to 250 nm.

In some embodiments the X and Y spacings are the same; in other embodiments the X and Y spacings are different.

A particularly suitable X spacing and Y spacing is about 200 nm (±5 nm).

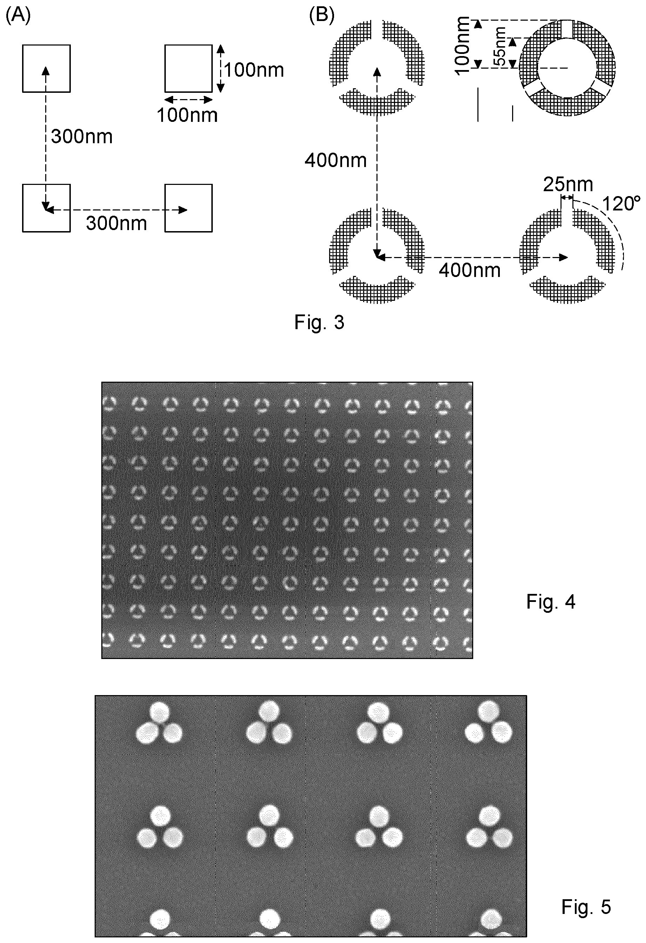

**Fig. 3** shows two exemplary designs of nanostructures and nanoarrays, with square nanostructures **(****Fig. 3(A)****)** and 'split ring' nanostructures **(****Fig. 3(B)). Figs. 4 and 5** are SEM images of example deposited nanoarrays, with 'split ring' nanostructures **(****Fig. 4****)** and 'trimer' nanostructures **(****Fig. 5****).**

### Fabrication

Devices of the present invention may be formed, in general terms, by known methods for deposition of ordered nanostructures on a surface. For example, one or more of photolithography and electron beam lithography may be useful techniques in such methods. General methods of this type are well known to those skilled in the art.

For example, the areas which will become nanophotonic regions may be defined on a (for example glass) substrate by photolithography. The areas which will become nanostructures, within those nanophotonic regions, may be defined by electron beam lithography.

In a suitable method of forming the nanophotonic regions (as optical windows), there may be the steps of applying a photoresist; patterning the resist to either expose the surface in the locations where a nanophotonic region is wanted or to expose the surface in the locations other than those where a nanophotonic region is wanted by exposure to light (for example UV light); and developing to remove the resist at either the exposed regions or the non-exposed regions. This leaves the desired patterned surface, which can then be further treated for example to chemically alter the surface other than the nanophotonic regions. Suitably the steps may be performed to leave photoresist protecting the nanophotonic regions (i.e. by removal of the resist at all other areas), such that on surface reaction (for example perfluorination) those regions are not reacted.

When the resist is then removed from the nanophotonic regions, they are distinct from the other regions by way of the chemical treatment (for example, they may be non-perfluorinated regions where the rest of the surface has been perfluorinated). This allows them to be addressed distinctly. Perfluorination of the areas other than those intended to form the nanophotonic regions is particularly suitable. It means that, when the solutions containing functionalising compounds are applied to the nanostructures which will be formed in the areas intended to form the nanophotonic regions, those solutions remain localised (the fluorination keeps them separated) without running into one another. Hence, by perfluorination of the spaces between the 'wells', higher density arrays can be employed.

In a suitable method for forming the nanostructures, there may be steps of applying a resist, patterning the resist to either expose the surface in the locations where a nanostructure is wanted or to expose the surface in the locations other than those where a nanostructure is wanted by exposure to an electron beam; and developing to remove the resist at either the exposed regions or the non-exposed regions.

For example, the resist may be exposed to facilitate its removal by a solvent; by exposing the areas to become nanostructures, the non-nanostructure parts of the surface are 'protected'. Metallisation can then form the desired nanostructures, with removal of the remaining resist leaving behind those nanostructures alone as metallised areas.

The metallisation which forms the nanostructures is preferably done by evaporation of at least gold onto the substrate, such that the eventually formed nanostructures comprise gold. It may optionally include evaporation of some other metal, for example titanium.

In particularly suitable embodiments, the gold is evaporated onto the surface to form a layer with a thickness of 20-80 nm, more suitably 40-60 nm and particularly suitably about 50 nm. That gold layer may in some embodiments be evaporated onto a titanium layer which itself has (first) been evaporated onto the substrate to a thickness of 1-10 nm, suitably 2-7 nm and particularly suitably about 5 nm.

Accordingly the present nanostructures preferably comprise gold, and optionally also comprise titanium; they may further preferably comprise a layer of titanium formed on the substrate and a layer of gold formed on the titanium layer.

After the nanostructures are formed, in the desired pattern of arrays and to the desired design, surface functionalisations are added. As described herein, these are important for the sensing activity of the devices. In some embodiments all arrays have a surface functionalisation; however, in other embodiments at least one array is left without a surface functionalisation (and is hence 'blank').

Surface functionalisations are added, broadly, by reacting (by contact) a solution containing the relevant functionalising compound with the nanophotonic region or array to be functionalised with that compound. In general each array has a particular functionalisation associated with it and each nanophotonic region within that array has the same functionalisation.

Each array is ideally designed so that its individual nanophotonic regions, or the array as a whole, is/are individually addressable, for example by a printer. This makes available a facile functionalisation by printing the functionalising compound in solution onto the desired nanophotonic region(s). In particular, this may be achieved by perfluorination of the space between the nanophotonic regions of the arrays, as explained above.

For example, solutions of the desired functionalising compounds may be made up by dissolving the functionalising compounds in a suitable solvent, for example water or ethanol. Those solutions can then be selectively printed (e.g. by dropwise application) to the arrays and nanophotonic regions as desired.

### Thermal Annealing

The present inventors have found that, in addition to the usual nanostructure fabrication techniques, a thermal annealing carried out before functionalisation can enhance the resonance quality of the nanostructures. These improvements make it easier to track small peak shifts.

The annealing step may slightly change the shape and size of the nanostructures; however, in general their design is unaffected.

The annealing step may itself be part of an annealing protocol include multiple heating and treating steps. An example protocol is set out below.

In suitable embodiments, the protocol includes an annealing step of treating the device at a temperature of 400 to 700°C (the annealing temperature) for a time of 60 to 1200 seconds. The temperature of this step may more preferably be 450 to 550°C. The time of this step may more preferably be 300 to 900 seconds.

The protocol may additionally include one or more purge steps, where the device is purged with an inert gas such as N₂. Each purge step may be for a time of 1 to 30 seconds.

The protocol may additionally include one or more vacuum steps, where the device is help in a vacuum. Each vacuum step may be for a time of 1 to 30 seconds.

Such purge and/or vacuum steps, where present, may be conducted at a temperature within the range 0°C to 100°C.

The protocol may also include periods of gradually raising temperature, which may be referred to as ramp steps. For example before the annealing step there may be a ramp step during which the temperature is raised from the ambient temperature to a temperature 100-200°C lower than the target annealing temperature. Such a ramp step may last from 5 to 100 seconds, for example 30 to 60 seconds.

One suitable protocol might be generalised as comprising at least:
1. a purge step;
2. a vacuum step;
3. a ramp step;
4. an annealing step;
5. a further vacuum step; and
6. a further purge step.

These annealing conditions are particularly suitable where the nanostructures comprises gold (Au).

### Functionalisation

Each surface functionalisation may individually be selected according to a desired sensing function; that is, according to the particular analyte(s) with which the surface functionalisation is intended to interact.

In the present invention, each of the first, second, third and fourth surface functionalisations are different. Other surface functionalisations may be the same as one of these, or different.

In some embodiments, each of the first, second, third, fourth, and fifth surface functionalisations (if present) are different. Other surface functionalisations may be the same as one of these, or different.

In some embodiments, each of the first, second, third, fourth, and fifth and sixth surface functionalisations (if present) are different. Other surface functionalisations may be the same as one of these, or different.

In some embodiments, each of the first, second, third, fourth, and fifth, sixth and seventh surface functionalisations (if present) are different. Other surface functionalisations may be the same as one of these, or different.

In some embodiments, each of the first, second, third, fourth, and fifth, sixth, seventh and eighth surface functionalisations (if present) are different. Other surface functionalisations may be the same as one of these, or different.

In some embodiments, each of the first, second, third, fourth, and fifth, sixth, seventh, eighth and ninth surface functionalisations (if present) are different. Other surface functionalisations may be the same as one of these, or different.

In some embodiments, each of the first, second, third, fourth, and fifth, sixth, seventh, eighth, ninth and tenth surface functionalisations (if present) are different. Other surface functionalisations may be the same as one of these, or different.

In some embodiments, each of the first, second, third, fourth, and fifth, sixth, seventh, eighth, ninth, tenth and eleventh surface functionalisations (if present) are different. Other surface functionalisations may be the same as one of these, or different.

In some embodiments, each of the first, second, third, fourth, and fifth, sixth, seventh, eighth, ninth, tenth, eleventh and twelfth surface functionalisations (if present) are different. Other surface functionalisations may be the same as one of these, or different.

In some embodiments, each of the first, second, third, fourth, and fifth, sixth, seventh, eighth, ninth, tenth, eleventh, twelfth and thirteenth surface functionalisations (if present) are different. Other surface functionalisations may be the same as one of these, or different.

In some embodiments, each of the first, second, third, fourth, and fifth, sixth, seventh, eighth, ninth, tenth, eleventh, twelfth, thirteenth and fourteenth surface functionalisations (if present) are different. Other surface functionalisations may be the same as one of these, or different.

In some embodiments, each of the first, second, third, fourth, and fifth, sixth, seventh, eighth, ninth, tenth, eleventh, twelfth, thirteenth, fourteenth and fifteenth surface functionalisations (if present) are different. Other surface functionalisations may be the same as one of these, or different.

Each surface functionalisation may suitably take the form of a chemical compound bound to the substrate. That compound may be chemically bonded to the substrate surface or otherwise held, for example by electrostatic interaction.

Where the substrate is selected appropriately, for example where it comprises gold (Au), surface functionalisations containing sulfur may suitably be used. The well-known affinity of sulfur for metals such as gold means that sulfur containing compounds such as thiols or disulfides are relatively easily deposited as surface functionalisations on the substrate. Such functionalising compounds are therefore preferably used in the present invention.

Conventionally it is thought that thiol compounds form a bond to gold, by 'chemisorbtion', with loss of hydrogen by an oxidative/reductive reaction leading to replacement of an R-S-H bond with an R-S-Au bond. This process of forming what is in effect a self-assembled monolayer is a well-known reaction. While recent research has suggested at least some physiosorbed character to the S-Au interaction, the exact nature of the binding of the compound to the substrate is not central to the present invention. Similarly, for disulfide compounds, an adsorption of the compound "intact" onto the gold surface, via multiple S-Au interactions but without cleavage of the S-S bond, may be involved.

Suitable chemical compounds for forming the surface functionalisations are, therefore, thiols R-S-H or disulfides R-S-S-R where the substrate is or comprises gold (Au). The corresponding surface functionalisations may exist as R-S-Au, R-S(Au)-S(Au)-R or similar.

It will be appreciated that 'thiols' as referred to herein may include more than one -SH group; similarly, 'disulfides' may include more than one -S-S- group.

For simplicity, in view of the complexity in trying to define the bonding interaction between the relevant compound and the substrate, surface functionalisations herein will be discussed in terms of the compounds used to form those functionalisations (so in the discussion above, for example, the thiols or disulfides). Those compounds used to form the surface functionalisations will herein be referred to as functionalising compounds.

It will be understood by those skilled in the art that the surface functionalisation itself may be derived or formed from such a functionalising compound by replacement of one or more R-S-H bonds in the thiol with a R-S-Au bond; or alternatively may be derived or formed by adsorption of the functionalising compound "intact" on the Au surface.

Accordingly, it will be recognised that each of the first, second, third and fourth surface functionalisations are derived from different functionalising compounds. Other surface functionalisations may be derived from the same functionalising compound as one of these, or different functionalising compounds.

In some embodiments of the invention, one or more of the surface functionalisations is derived from a functionalising compound that is a thiol. In some embodiments, one or more of the surface functionalisations is derived from a functionalising compound that is a disulfide. In some embodiments, each surface functionalisation is derived from a functionalising compound that is a thiol or a disulfide.

Where the functionalising compound is a thiol, in some embodiments it may be a monothiol or a dithiol.

In some embodiments, one or more the surface functionalisations (and preferably each surface functionalisation) is derived from a functionalising compound which has a formula individually selected from the following (I) to (VI):

| | |
|---|---|
| (I) | 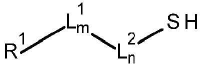 |

wherein:
m is 0 or 1;
n is 0 or 1;
R¹ is selected from the group consisting of C₁₋₂₀ straight or branched alkyl, and 5-10 membered cyclic, and is optionally substituted;
L¹, if present, is selected from the group consisting of C₁₋₂₀ straight or branched alkylene, C₂₋₁₀ alkenylene, and 5-10 membered cyclic, and is optionally substituted; and
L², if present, is selected from the group consisting of C₁₋₂₀ straight or branched alkylene, C₂₋₁₀ alkenylene, and 5-10 membered cyclic, and is optionally substituted;
with the condition that, where m = 1 and n = 1, if either L¹ or L² is alkenylene, then the other of L¹ and L² is not alkenylene;

| | |
|---|---|
| (II) | 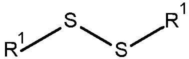 |

wherein: each R¹ is independently selected from the group consisting of C₁₋₂₀ straight or branched alkyl, and 5-10 membered cyclic, and is optionally substituted;
or alternatively the two R¹ groups are linked to form a 5-10 membered heterocyclic which is optionally substituted;

| | |
|---|---|
| (III) | 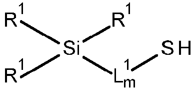 |

wherein:
m is 0 or 1;
each R¹ is the same or different and is selected from the group consisting of C₁₋₂₀ straight or branched alkyl, and 5-10 membered cyclic, and is optionally substituted; and
L¹, if present, is selected from the group consisting of C₁₋₂₀ straight or branched alkylene, C₂₋₁₀ alkenylene, and 5-10 membered cyclic, and is optionally substituted;

| | |
|---|---|
| (IV) | 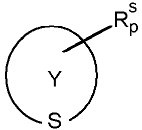 |

wherein:
Y represents a 5-10 membered heterocyclic system containing exactly one S atom as the sole heteroatom;
p is 0, 1, 2 or 3;
and each R^{s} is independently selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in Y is replaced by -C(NH)-), =O (that is, one carbon atom in Y is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl;

| | |
|---|---|
| (V) | 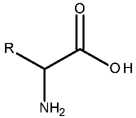 |

wherein R is an amino acid side chain including at least one -SH group;
and

| | |
|---|---|
| (VI) | 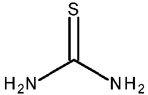 |

As a reactant for functionalisation, the functionalising compound may suitably be provided as a salt, for example a sodium salt. Such a salt might, for example, have the formula:

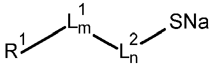

Another suitable salt is a hydrochloride salt, which might, for example, have the formula:

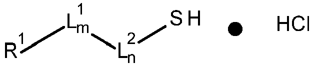

Of course it will be recognised that while these illustrations are based on (I), salts of any of the above (I) to (VI) might be used.

It will be understood that each functionalising compound can be chosen separately, and each preference or embodiment set out below can apply individually to each functionalising compound. So, the various functionalising compounds used to provide the various different functionalisations may be chosen separately according to different ones of the embodiments and preferences, and combinations thereof, set out here.

### R¹ - primary structure

R¹ is selected from the group consisting of C₁₋₂₀ straight or branched alkyl, C₅₋₇ cycloalkyl, and 5-10 membered cyclic.

Where R¹ is C₁₋₂₀ straight or branched alkyl, where m = 0 and n = 0, it may suitably be C₂₋₁₅ straight or branched alkyl, in particular C₂₋₁₂ straight or branched alkyl. More suitably it may be C₂, C₄, C₆, C₈, C₁₀ or C₁₂ straight or branched alkyl. Where m = 1 and/or n = 1, it may suitably be C₁₋₆ straight or branched alkyl, more suitably methyl, ethyl, n-propyl, iso-propyl or tert-butyl.

Particular examples of R¹ as a straight alkyl include methyl, ethyl, n-propyl, n-butyl, n-pentyl, n-hexyl, n-heptyl, n-octyl, n-nonyl, n-decyl, n-undecyl, n-dodecyl, n-hexadecyl or n-octadecyl.

Where R¹ is a branched alkyl group, any isomer may be suitable. For example, as a branched C₅ alkyl group, R¹ may be n-pentyl, 2-methylbutyl, or 2,2-dimethylpropyl.

Particular examples of R¹ as a branched alkyl include 2-methylpropyl, tert-butyl, 2-methylbutyl, 3-methylbutyl and 2-ethylhexyl.

Where R¹ is a 5-10 membered cyclic structure, it may be non-aromatic or aromatic. It may be carbocyclic or heterocyclic. It may contain a single ring, or multiple fused rings.

For example, it may be an aromatic carbocyclic structure such as phenyl or naphthyl. It may be a non-aromatic carbocyclic structure such as cyclopentyl, cyclohexyl or cycloheptyl.

Alternatively it may be a heterocyclic structure containing one, two, three or four hetero atoms. Those hetero atoms may each be individually selected from, for example, O, S and N.

Suitable examples include pyrrolidinyl, oxolanyl, thiolanyl, pyrrolyl, furanyl, thiophenyl, piperidinyl, oxanyl, thianyl, pyridinyl, pyranyl, thiopyranyl, azepanyl, oxepanyl, thiepanyl, azepinyl, oxepinyl, thiepinyl, imidazolidyl, imidazolyl, pyrazolidyl, pyrazolyl, oxathiolidinyl, oxathiolyl, isothiolidinyl, isoxathiolyl, oxazolidinyl, oxazolyl, isoxazolidinyl, isoxazolyl, thiazolidinyl, thiazolyl, isothiazolidinyl, isothiazolyl, dioxolanyl, dithiolanyl, triazolyl, piperidinyl, pyridinyl, oxanyl, pyranyl, thianyl, thiopyranyl, diazinanyl, diazinyl, morpholino, oxazinyl, thiomorpholino, thiazinyl, dioxanyl, dioxinyl, dithianyl, dithiinyl, triazinanyl, triazinyl, trioxanyl, trithianyl, tetrazinyl, benzothiazolyl, benzimidazolyl, benzoxazolyl, benzisoxazolyl and indolyl.

Particularly suitable cyclic R¹ groups include cyclohexyl, phenyl, naphthyl, furanyl, triazolyl, benzoxazolyl, benzimidazolyl, pyridinyl, oxanyl, imidazolyl and triazinyl (preferably 1,3,5-triazinyl).

More particularly suitable cyclic R¹ groups include phenyl, triazolyl and furanyl.

All of the above mentioned options for the primary structure of R¹ may be optionally substituted as described herein.

In formula (II), it may be preferred that the two R¹ groups are the same. They may for example each be optionally substituted phenyl.

In formula (III), it may be preferred that the three R¹ groups are the same. They may preferably be selected from methyl, ethyl, n-propyl, iso-propyl, tert-butyl, and phenyl.

### R¹ - optional substitution

R¹ is optionally substituted. That optional substitution may be, for example, one or more substitutions with group(s) R^{s} independently selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in R¹ is replaced by -C(NH)-), =O (that is, one carbon atom in R¹ is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

Where R¹ is substituted, it may be suitably substituted with one or more of the groups R^{s} mentioned above; in particular with one, two, three or four substitutions each independently selected from those R^{s} mentioned above.

In some embodiments, R¹ maybe be substituted with exactly one group or exactly two groups selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in R¹ is replaced by -C(NH)-), =O (that is, one carbon atom in R¹ is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

In some embodiments R¹ maybe be substituted with exactly one group or exactly two groups selected from the list consisting of COOH, NO₂, B(OH)₂, Cl and NH₂.

In some embodiments, R¹ may be substituted with exactly one, exactly two, exactly three or exactly four groups selected from halogen (for example, F, Br, or CI).

In some embodiments, particularly where R¹ is C₁₋₂₀ straight or branched alkyl, it may be that every hydrogen atoms of the 'alkyl' chain is replaced with halogen (for example, F, Br, or Cl). That is, R¹ is CₓHal₂ₓ₊₂, wherein x is 1-20 and Hal is F, Br or Cl. This particularly applies in formula (I) where the sum of n and m is 1 and L¹ or L² (whichever is present) is a C₁₋₆ straight or branched alkylene.

Where more than one halogen substitution is present on R¹, they may be the same or different; suitably, each halogen substitution is the same (that is, all F, or all Cl, or all Br).

Where R¹ is substituted with CH₂SH or SH, it may be suitable that it is substituted with exactly one SH group (this making the compound of Formula (I) a dithiol). Other substitutions may also be present in such embodiments.

### Linked R¹-R¹

In the above mentioned (II), the two R¹ groups may be linked to form a 5-10 membered heterocyclic, which is optionally substituted. In some embodiments they may be linked to form a 5 or 6 membered heterocyclic. The heterocyclic may be saturated or unsaturated; in preferred embodiments it is saturated.

The heterocyclic structure is optionally substituted. In preferred embodiments it is substituted. The substituent(s) are each individually C₁₋₂₀ straight or branched alkyl, and may themselves be further optionally substituted with a substituent chosen from the same optional substituents R^{s} discussed for R¹ in the section above. In particularly preferred embodiments the heterocyclic structure is substituted with a single C₁₋₂₀ straight or branched alkyl group, which is itself substituted with a group selected from halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in R¹ is replaced by -C(NH)-), =O (that is, one carbon atom in R¹ is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl. Of these -COOH is particularly preferred.

In particularly preferred embodiments, the optional substituent in these linked R¹-R¹ embodiments is ortho to one of the sulfur atoms; that is, the substitution is on a carbon atom adjacent to a sulfur atom in the cyclic structure.

### L¹ - primary structure

L¹, if present in formula (I) or (III), is selected from the group consisting of C₁₋₂₀ straight or branched alkylene, C₂₋₁₀ alkenylene, and 5-10 membered cyclic, and is optionally substituted.

Where L¹ is C₁₋₂₀ straight or branched alkylene, it may suitably be C₂₋₁₅ straight or branched alkylene, in particular C₂₋₁₂ straight or branched alkylene. More suitably it may be C₂, C₄, C₆, C₈, C₁₀ or C₁₂ straight or branched alkylene. It may suitably be C₁₋₆ straight or branched alkylene, more suitably methylene, ethylene, n-propylene, iso-propylene or tert-butylene.

Where L¹ is C₁₋₂₀ straight or branched alkylene as set out above, it may be linked to Si (in formula (III), R¹ (in formula (I)), L² (if present in formula (I)) or the SH group at any point along its chain length. For example, in formula (I), where m=1, L¹ is C₂ alkylene and n=0, R¹ and the SH group may be attached to the same carbon atom or different carbon atoms. Where L¹ is C₃ alkylene, R¹ and the SH group may be attached to the same carbon atom; or may be attached to the alpha and beta carbon atoms, or may be attached to the alpha and gamma carbon atoms.

Particular examples of L¹ as a straight alkylene include methylene, ethylene, n-propylene, n-butylene, n-pentylene, n-hexylene, n-heptylene, n-octylene, n-nonylene, n-decylene, n-undecylene, n-dodecylene, n-hexadecylene or n-octadecylene.

Where L¹ is a branched alkylene group, any isomer may be suitable. For example, as a branched C₅ alkylene group, L¹ may be n-pentylene, 2-methylbutylene, or 2,2-dimethylpropylene.

Particular examples of L¹ as a branched alkylene include 2-methylpropylene, tert-butylene, 2-methylbutylene, 3-methylbutylene and 2-ethylhexylene.

Where L¹ is a 5-10 membered cyclic structure, it may be non-aromatic or aromatic. It may be carbocyclic or heterocyclic. It may contain a single ring, or multiple fused rings.

For example, it may be an aromatic carbocyclic structure such as phenylene or naphthylene. It may be a non-aromatic carbocyclic structure such as cyclopentylene, cyclohexylene or cycloheptylene.

Alternatively it may be a heterocyclic structure containing one, two, three or four hetero atoms. Those hetero atoms may each be individually selected from, for example, O, S and N.

Suitable examples include pyrrolidinylene, oxolanylene, thiolanylene, pyrrolylene, furanylene, thiophenylene, piperidinylene, oxanylene, thianylene, pyridinylene, pyranylene, thiopyranylene, azepanylene, oxepanylene, thiepanylene, azepinylene, oxepinylene, thiepinylene, imidazolidylene, imidazolylene, pyrazolidylene, pyrazolylene, oxathiolidinylene, oxathiolylene, isothiolidinylene, isoxathiolylene, oxazolidinylene, oxazolylene, isoxazolidinylene, isoxazolylene, thiazolidinylene, thiazolylene, isothiazolidinylene, isothiazolylene, dioxolanylene, dithiolanylene, triazolylene, piperidinylene, pyridinylene, oxanylene, pyranylene, thianylene, thiopyranylene, diazinanylene, diazinylene, morpholino-ene, oxazinylene, thiomorpholino-ene, thiazinylene, dioxanylene, dioxinylene, dithianylene, dithiinylene, triazinanylene, triazinylene, trioxanylene, trithianylene, tetrazinylene, benzothiazolylene, benzimidazolylene, benzoxazolylene, benzisoxazolylene and indolylene.

Particularly suitable cyclic L¹ groups include cyclohexylene, phenylene, naphthylene, furanylene, triazolylene, benzoxazolylene, benzimidazolylene, pyridinylene, oxanylene, imidazolylene and triazinylene (preferably 1,3,5-triazinylene).

Where L¹ has a cyclic structure, it may be linked to Si (in formula (III), R¹ (in formula (I)), L² (if present in formula (I)) or the SH group at any point around that cyclic structure.

For example, in formula (I), where m=1, L¹ is phenylene, and n=0, R¹ and the SH group may be positions ortho, meta or para to one another.

### L¹ - optional substitution

L¹, if present, is optionally substituted. That optional substitution may be, for example, one or more substitutions with group(s) R^{s} independently selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in L¹ is replaced by -C(NH)-), =O (that is, one carbon atom in L¹ is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

Where L¹ is substituted, it may be suitably substituted with one or more of the groups mentioned above; in particular with one, two, three or four substitutions each independently selected from those mentioned above.

In some embodiments, L¹ maybe be substituted with exactly one group or exactly two groups selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, SH, =NH (that is, one carbon atom in L¹ is replaced by - C(NH)-), =O (that is, one carbon atom in L¹ is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

In some embodiments, L¹ may be substituted with exactly one, exactly two, exactly three or exactly four groups selected from halogen (for example, F, Br, or CI).

In some embodiments, particularly where L¹ is C₁₋₂₀ straight or branched alkylene, it may be that every hydrogen atoms of the 'alkyl' chain is replaced with halogen (for example, F, Br, or CI). That is, L¹ is CₓHal₂ₓ, wherein x is 1-20 and Hal is F, Br or Cl.

Where more than one halogen substitution is present on L¹, they may be the same or different; suitably, each halogen substitution is the same (that is, all F, or all Cl, or all Br).

### L² - primary structure

L², if present in formula (I), is selected from the group consisting of C₁₋₂₀ straight or branched alkylene, C₂₋₁₀ alkenylene, and 5-10 membered cyclic, and is optionally substituted.

Where L² is C₁₋₂₀ straight or branched alkylene, it may suitably be C₂₋₁₅ straight or branched alkylene, in particular C₂₋₁₂ straight or branched alkylene. More suitably it may be C₂, C₄, C₆, C₈, C₁₀ or C₁₂ straight or branched alkylene. It may suitably be C₁₋₆ straight or branched alkylene, more suitably methylene, ethylene, n-propylene, iso-propylene or tert-butylene.

Where L² is C₁₋₂₀ straight or branched alkylene as set out above, it may be linked R¹, L² (if present) or the SH group at any point along its chain length. For example, in formula (I), where n=1, L² is C₂ alkylene and m=0, R¹ and the SH group may be attached to the same carbon atom or different carbon atoms. Where L² is C₃ alkylene, R¹ and the SH group may be attached to the same carbon atom; or may be attached to the alpha and beta carbon atoms, or may be attached to the alpha and gamma carbon atoms.

Particular examples of L² as a straight alkylene include methylene, ethylene, n-propylene, n-butylene, n-pentylene, n-hexylene, n-heptylene, n-octylene, n-nonylene, n-decylene, n-undecylene, n-dodecylene, n-hexadecylene or n-octadecylene.

Where L² is a branched alkylene group, any isomer may be suitable. For example, as a branched C₅ alkylene group, L² may be n-pentylene, 2-methylbutylene, or 2,2-dimethylpropylene.

Particular examples of L² as a branched alkylene include 2-methylpropylene, tert-butylene, 2-methylbutylene, 3-methylbutylene and 2-ethylhexylene.

Where L² is a 5-10 membered cyclic structure, it may be non-aromatic or aromatic. It may be carbocyclic or heterocyclic. It may contain a single ring, or multiple fused rings.

For example, it may be an aromatic carbocyclic structure such as phenylene or naphthylene. It may be a non-aromatic carbocyclic structure such as cyclopentylene, cyclohexylene or cycloheptylene.

Alternatively it may be a heterocyclic structure containing one, two, three or four hetero atoms. Those hetero atoms may each be individually selected from, for example, O, S and N.

Suitable examples include pyrrolidinylene, oxolanylene, thiolanylene, pyrrolylene, furanylene, thiophenylene, piperidinylene, oxanylene, thianylene, pyridinylene, pyranylene, thiopyranylene, azepanylene, oxepanylene, thiepanylene, azepinylene, oxepinylene, thiepinylene, imidazolidylene, imidazolylene, pyrazolidylene, pyrazolylene, oxathiolidinylene, oxathiolylene, isothiolidinylene, isoxathiolylene, oxazolidinylene, oxazolylene, isoxazolidinylene, isoxazolylene, thiazolidinylene, thiazolylene, isothiazolidinylene, isothiazolylene, dioxolanylene, dithiolanylene, triazolylene, piperidinylene, pyridinylene, oxanylene, pyranylene, thianylene, thiopyranylene, diazinanylene, diazinylene, morpholino-ene, oxazinylene, thiomorpholino-ene, thiazinylene, dioxanylene, dioxinylene, dithianylene, dithiinylene, triazinanylene, triazinylene, trioxanylene, trithianylene, tetrazinylene, benzothiazolylene, benzimidazolylene, benzoxazolylene, benzisoxazolylene and indolylene.

Particularly suitable cyclic L² groups include cyclohexylene, phenylene, naphthylene, furanylene, triazolylene, benzoxazolylene, benzimidazolylene, pyridinylene, oxanylene, imidazolylene and triazinylene (preferably 1,3,5-triazinylene).

Where L² has a cyclic structure, it may be linked to R¹, L¹ (if present) or the SH group at any point around that cyclic structure.

For example, in formula (I), where n=1, L² is phenylene, and m=0, R¹ and the SH group may be positions ortho, meta or para to one another.

### L² - optional substitution

L², if present, is optionally substituted. That optional substitution may be, for example, one or more substitutions with group(s) R^{s} independently selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, CH₂SH, SH, =NH (that is, one carbon atom in L² is replaced by -C(NH)-), =O (that is, one carbon atom in L² is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

Where L² is substituted, it may be suitably substituted with one or more of the groups mentioned above; in particular with one, two, three or four substitutions each independently selected from those mentioned above.

In some embodiments, L² maybe be substituted with exactly one group or exactly two groups selected from the list consisting of C₁₋₃ straight alkyl, halogen (for example, F, Br, or CI), NH₂, NH(C₁₋₆ straight alkyl), N(C₁₋₆ straight alkyl)₂, COOH, OH, NO₂, SH, =NH (that is, one carbon atom in L² is replaced by - C(NH)-), =O (that is, one carbon atom in L² is replaced by -C(O)-), OR², B(OH)₂, OP(O)(OH)₂ and NHC(O)CH₃;
wherein each R² is independently selected from CF₃, C₁₋₆ alkyl, C(O)C₁₋₆ alkyl and C₁₋₆ alkenyl-O-C₁₋₆ alkyl.

In some embodiments, L² may be substituted with exactly one, exactly two, exactly three or exactly four groups selected from halogen (for example, F, Br, or CI).

In some embodiments, particularly where L² is C₁₋₂₀ straight or branched alkylene, it may be that every hydrogen atoms of the 'alkyl' chain is replaced with halogen (for example, F, Br, or CI). That is, L² is CₓHal₂ₓ, wherein x is 1-20 and Hal is F, Br or Cl.

Where more than one halogen substitution is present on L², they may be the same or different; suitably, each halogen substitution is the same (that is, all F, or all Cl or all Br).

### Y

Y represents a 5-10 membered heterocyclic system containing exactly one S atom as the sole heteroatom. It may suitably be a 5, 6 or 7 membered heterocyclic ring containing exactly one S atom as the sole heteroatom.

In some embodiments, Y is saturated.

In preferred embodiments Y is tetrahydrothiophene, thiane or thiepane.

### R

R represents a side chain in the amino acid compounds of formula (VI). Its structure is therefore not particularly limited from a chemical perspective. Suitable side chains are well known in the art. The only requirement in the present invention is that the side chain includes at least on -SH group, to facilitate the functionalisation reaction as described above.

Suitable side chains include, for example, those which can be defined in terms of the groups mentioned above as -L²ₙ-L¹ₘ-R¹.

Particularly suitable side chains include:
C₁₋₂₀ straight or branched alkyl substituted with -SH, for example -CH₂-SH, and
a C₁₋₂₀ straight or branched alkyl substituted with -SH or -CH₂SH and with OH, wherein one or more of the carbon atoms in the straight or branched alkyl is replaced with NH and one or more of the carbon atoms in the straight or branched alkyl is replaced with C(O).

In some embodiments, R is such that formula (VI) is glutathione, preferably, L-glutathione:

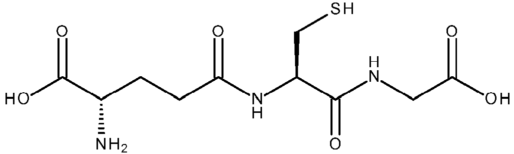

In some embodiments, R is such that formula (VI) is cysteine, in particular L-cysteine or D-cysteine:

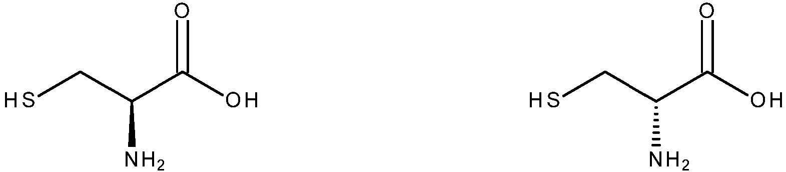

### m, n and p

m, n and p represent integers indicating the numbers of times a certain group appears in a given formula.

m is 0 or 1. In some embodiments it is 0. In some embodiments it is 1.

n is 0 or 1. In some embodiments it is 0. In some embodiments it is 1.

p is 0, 1, 2 or 3. In some embodiments it is 0. In some embodiments it is 1. In some embodiments it is 2. In some embodiments it is 3.

In formula (I), the sum of m and n is 0, 1 or 2. In some embodiments the sum of m and n is 0. In some embodiments the sum of m and n is 1. In some embodiments the sum of m and n is 2.

In some preferred embodiments, where m= 0 and n = 0, in formula (I) the SH group is bonded directly to R¹.

In some preferred embodiments, where m = 0, in formula (III) the SH group is bonded directly to Si.

In some embodiments, where m =1 and n = 1, L¹ and L² are the same.

### Particularly Suitable Functionalisations

The present inventors have found that, for the thiols or disulfides functionalizing the substrate, the non-thiol/disulfide part of the molecule can play an important function in the selectivity of sensing. Accordingly, the non-thiol/disulfide part of the molecule may be suitably chosen depending on the target analyte(s) whose presence is to be sensed in a particular use case.

For example, one or more of the functionalising compounds (and hence functionalisations) may suitably include a group of the following type, for the reason mentioned:

| | |
|---|---|
| Carboxylic acid (COOH), alcohol (OH) or ketone (CO) | Acid character |
| Amine (NH₂) or other nitrogen containing group (e.g. amide) | Basic character |
| Aliphatic group | Hydrophobic character |
| Aromatic ring | To detect other aromatic compounds via pi-pi interaction |
| Nitro group | Reduced in the presence of some sugars; electron deficient |
| Boronic acid group | Binds diols (e.g. in sugars) to form cyclic borate esters (labile covalency) |

That is, in some embodiments, each surface functionalisation is derived from a functionalising compound that is a thiol or a disulfide and that contains at least one of a carboxylic acid group COOH, an alcohol group OH, a ketone group CO, an amine group NH₂, an amide group, an aliphatic group, an aromatic group, a nitro group NO₂ or a boronic acid group B(OH)₂.

In some embodiments, the surface functionalisations on the substrate are selected such that, amongst their number, at least an alcohol group OH; an amine group NH₂; an aliphatic group; an aromatic group; and a halogen group (F, Cl or Br) are present.

In some embodiments, the surface functionalisations on the substrate are selected such that, amongst their number, at least a carboxylic acid group COOH; an alcohol group OH; an amine group NH₂; an aliphatic group; an aromatic group; and a halogen group (F, Cl or Br) are present.

In some embodiments, the surface functionalisations on the substrate are selected such that, amongst their number, at least a carboxylic acid group COOH; an alcohol group OH; an amine group NH₂; an aliphatic group; an aromatic group; a nitro group NO₂; and a halogen group (F, Cl or Br) are present.

In some embodiments, the surface functionalisations on the substrate are selected such that, amongst their number, at least one of each of: a carboxylic acid group COOH, an alcohol group OH, or a ketone group CO; an amine group NH₂; an amide group; an aliphatic group; an aromatic group; a nitro group NO₂; a boronic acid group B(OH)₂; and a halogen group (F, Cl or Br) is present.

Furthermore, some variety in general functionalisation may be advantageous. In view of that, it may be preferred that the surface functionalisations on the substrate are selected such that, amongst their number, at least one is derived from a functionalising compound according to the above formula (I). Preferably amongst their number at least one is derived from a functionalising compound according to the above formula (I) and at least one is derived from a functionalising compound according to the above formula (II). Preferably amongst their number at least one is derived from a functionalising compound according to the above formula (I), at least one is derived from a functionalising compound according to the above formula (II) and at least one is derived from a functionalising compound according to the above formula (V).

These preferences can of course apply individually in combination.

### Example Suitable Functionalisations

In some embodiments, at least one of the surface functionalisations (and suitably each surface functionalisation) is derived from a functionalising compound which is selected from: 1-propanethiol, 1-butanethiol, 1-pentanethiol, 1-hexanethiol, 1-heptanethiol, 1-octanethiol, 1-nonanethiol, 1-decanethiol, 1-undecanethiol, 1-dodecanethiol, 1-hexadecanethiol, 1-octadecanethiol, 2-methyl-1-propanethiol, 2-methyl-2-propanethiol, cyclohexanethiol, 2-methyl-1-butanethiol, 3-methyl-1-butanethiol, 2-ethylhexanethiol, 2-methyl-1-butanethiol, tert-dodecanethiol, 2-ethylhexanethiol, thiophenol, benzyl mercaptan, 1-napthalenethiol, 2-naphthalenethiol, 4-biphenylthiol, 1,1',4',1"-terphenyl-4-thiol, 4-methylbenzenethiol, 2-phenylethanethiol, 4-isopropylbenzenethiol, 4-tert-butylbenzenethiol, 1-phenylethanethiol, 1,4-benzenedimethanethiol, 1,4-butanedithiol, 1,6-hexanedithiol, 1,8-octanedithiol, 1,10-decanedithiol, p-terphenyl-4,4"-dithiol, 1,2-ethanedithiol, biphenyl-4,4'-dithiol, 1,16-hexadecanedithiol, benzene-1,3-dithiol, benzene-1,4-dithiol, 4,4'-bis(mercaptomethyl)biphenyl, 1,2-benzenedimethanethiol, benzene-1,3-dithiol, 4,4'-dimercaptostilbene, 4-bromobenzenethiol, 2-bromobenzenethiol, 3-bromothiophenol, 4-bromobenzyl mercaptan, 3-bromobenzyl mercaptan, 2-bromobenzyl mercaptan, 4-chlorobenzenethiol, 3-chlorothiophenol, 3-chloro-1-propanethiol, 2,4-dichlorobenzenethiol 3,4-dichlorobenzenethiol, 2,5-dichlorobenzenethiol, 2-chlorothiophenol, 3,4-difluorothiophenol, 4-fluorobenzyl mercaptan, 4-trifluoromethylbenzyl mercaptan, 2,4-difluorothiophenol, 4-fluorothiophenol, 1H,1H,2H,2H-perfluorodecanethiol, tetrafluorothiophenol, 2,3,5,6-tetrafluorobenzenethiol, 2-amino-4-chlorobenzenethiol, 3-(trifluoromethoxy)thiophenol, heptadecafluoro-1-decanethiol, 4-mercaptobenzoic acid, 6,8-thioctic acid, 3-mercaptopropionic acid, 6-mercaptohexanoic acid, 3-mercaptoisobutyric acid, 3-mercaptobenzoic acid, thioacetic acid, 11-mercaptoundecanoic acid, 4-mercaptophenylacetic acid, 4-(mercaptomethyl)benzoic acid, thioglycolic acid, 4-mercaptobutyric acid, 6-mercaptohexanoic acid, mercaptosucccinic acid, meso-2,3-dimercaptosuccinic acid, 6-mercaptopyridine-3-carboxylic acid, 3-mercaptohexyl hexanoate, 1-thio-β-D-glucose sodium salt, 6-mercapto-1-hexanol, 4-mercapto-4-methylpentan-2-ol, 3-mercapto-1-hexanol, 4-mercaptophenol, 1-thioglycerol, 2,3-dimercapto-1-propanol, 2-mercaptoethanol, DL-dithiothreitol, 3-mercapto-1-propanol, 4-methyl-4-mercaptopentan-2-one, 2-furanmethanethiol, 2-(2-methoxyethoxy)ethanethiol, 3-ethoxythiophenol, 4-methoxybenzenethiol, 4-methoxybenzyl mercaptan, 4-nitrothiophenol, (4-nitrobenzyl)mercaptan, 5,5'-dithiobis(2-nitrobenzoic acid), 3-aminothiophenol, 4-aminothiophenol, 2-aminothiophenol, thiourea, 2-(butylamino)ethanethiol, 2-diethylaminoethanethiol hydrochloride, 3-amino-5-mercapto-1,2,4-triazole, 4-ethyl-4H-1,2,4-triazole-3-thiol, 2-(5-sulfanyl-4H-1,2,4-triazol-3-yl)phenol, 2-mercaptobenzoxazole, 4-mercaptopyridine, 2-mercaptopyridine, 2-mercaptobenzimidazole, 1-(11-mercaptoundecyl)imidazole, 4-acetamidothiophenol, 2-iminothiolane hydrochloride, 2-(trimethylsilyl)ethanethiol, triphenylsilanethiol, triisopropylsilanethiol, 4-nitrophenyldisulfide, p-tolyl disulfide, L-cysteine, D-cysteine, L-glutathione, Cysteamine, 4-mercaptophenylboronic acid, 3-mercaptophenylboronic acid, 11-mercaptoundecylphosphoric acid, and trithiocyanuric acid.

### Specific Functionalisations

For example, in a sensing device suitable for use in analysing beer, at least one of the surface functionalisations may suitably be derived from a functionalising compound which is 3,4-dichlorothiophenol (DTP).

A further exemplary embodiment of the present sensing device comprises first, second, third and fourth surface functionalisations (and, of course, corresponding arrays), respectively derived from functionalising compounds which are 1-Dodecanethiol (DDT), 6-mercapto-1-hexanol (MHOH), 3-amino-5-mercapto-1,2,4-triazole (AMT) and 3,4-dichlorothiophenol (DTP).

A further exemplary embodiment of the present sensing device comprises first, second, third, fourth and fifth surface functionalisations (and, of course, corresponding arrays), respectively derived from functionalising compounds which are 1-Dodecanethiol (DDT), 4-mercaptobenzoic acid (MBA), 6-mercapto-1-hexanol (MHOH), 3-amino-5-mercapto-1,2,4-triazole (AMT) and 3,4-dichlorothiophenol (DTP).

A further exemplary embodiment of the present sensing device comprises first, second, third, fourth, fifth and sixth surface functionalisations (and, of course, corresponding arrays), respectively derived from functionalising compounds which are 1H,1H,2H,2H-perfluorodecanethiol (PFDT), 1-Dodecanethiol (DDT), 4-mercaptobenzoic acid (MBA), 6-mercapto-1-hexanol (MHOH), 3-amino-5-mercapto-1,2,4-triazole (AMT) and 3,4-dichlorothiophenol (DTP).

A further exemplary embodiment of the present sensing device comprises first, second, third, fourth, fifth, sixth and seventh surface functionalisations (and, of course, corresponding arrays), respectively derived from functionalising compounds which are 1H,1H,2H,2H-perfluorodecanethiol (PFDT), 1-Dodecanethiol (DDT), 4-mercaptobenzoic acid (MBA), 6-mercapto-1-hexanol (MHOH), 3-amino-5-mercapto-1,2,4-triazole (AMT), 4-nitrothiophenol (NTP) and 3,4-dichlorothiophenol (DTP).

A further exemplary embodiment of the present sensing device comprises first, second, third, fourth, fifth, sixth, seventh, eighth, ninth, tenth, eleventh, twelfth, thirteenth, fourteenth and fifteenth surface functionalisations (and, of course, corresponding arrays), respectively derived from functionalising compounds which are 1H,1H,2H,2H-perfluorodecanethiol (PFDT), 1-Dodecanethiol (DDT), 4-mercaptobenzoic acid (MBA), 6-mercapto-1-hexanol (MHOH), 3-amino-5-mercapto-1,2,4-triazole (AMT), 4-nitrothiophenol (NTP), 3,4-dichlorothiophenol (DTP), 1-Octanethiol (OT), 3-mercaptopropionic acid (MPA), 11-mercaptoundecanoic acid (MUA), 2-Phenyletanethiol (PET), 4-mercaptophenylboronic acid (MPBA), 2-furanmethanethiol (FMT), 5,5'-Dithiobis(2-nitrobenzoic acid) (NBA) and L-glutathione (GLU).

As set out herein, this further exemplary embodiment may suitably also comprise a further (here, sixteenth) array which is left 'pristine' or 'blank', that is, without surface functionalisation.

### Examples

### Device Fabrication

Firstly, the optical windows within which the nanostructures are to be formed were created.

A glass sample was dehydrated on a hotplate at 100°C for 10 minutes followed by spinning of a Microposit S1818 resist (4000 RPM, 30 seconds). The sample is then baked on a hotplate at 115°C for 3 minutes. The resist was then patterned in a grid (to form 16 arrays, each corresponding to a row of 24 nanophotonic regions, with 4.5mm spacing between rows and between regions in a given row) via UV exposure through a chrome photomask using a Suss MA/BA8 Optical Mask Aligner, at a constant intensity of exposure at 90 mJ/cm² (with alignment). The sample was then developed in Microposit MF319 developer for 2 minutes followed by soaking the sample in reverse osmosis water to stop the development and N₂-compressed-air dried. The sample was then treated with O₂ plasma (PlasmaFab RF Barrel Asher) at 110 W for 1 minute to both descum and active the surface. Then the sample was placed in a reaction chamber with 1H,1H,2H,2H-Perfluorodecyltrimethoxysilane. The chamber was then heated on a hotplate at 90°C, for 30 minutes, causing the exposed surface of the substrate to be perfluorinated.

The nanophotonic regions (currently merely wells) of the sample were protected from perfluorination by the pillar of photoresist.

The sample was then taken out and placed in an N₂ oven at 90°C for 30-60 minutes. Lift-off of the resist and metal on top of the resist was completed by soaking the sample in an acetone bath at 50°C for >15 minutes, followed by pipette agitation until all the metal pillars are lifted off. The sample was then transferred to fresh acetone, then isopropyl alcohol and then N₂-compressed-air dried.

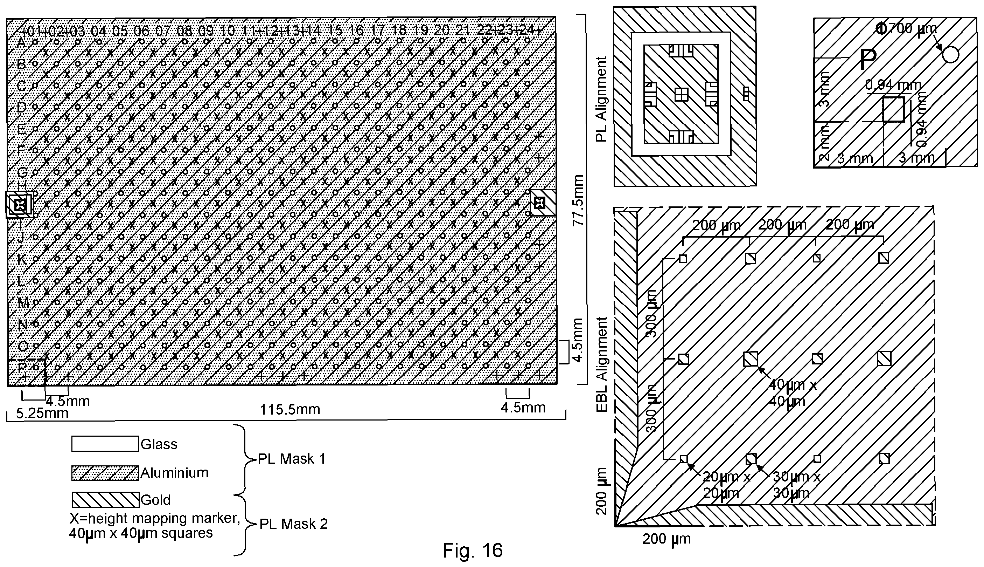

Secondly, in order to accurately 'target' the wells for nanostructure deposition, alignment markers are defined by a second photolithography step. The specific design for the masking and alignment is shown in Fig. 16, in which white dots represent the wells/nanophotonic regions.

The sample was patterned according to this design, aligned to the pattern in the above mentioned optical window creation step. They were then blown with an N₂ gun and spin coated with Microposit S1818 photoresist at 4000 RPM for 30 seconds. The samples were then baked on a hot plate at 115°C for 3 minutes, followed by UV exposure through a chrome photomask using a Suss MA/BA8 Optical Mask Aligner, at a constant intensity of exposure at 90 mJ/cm² (with alignment). The sample was then developed in Microposit MF 319 developer for 90 seconds, then submerged in running deioning or reverse osmosis water to stop development. Finally it was dried under N₂.

The sample was then treated with O₂ plasma (Asher PlasmaFab RF Barrel) at 110 W for 60 seconds.

It was next metallised (Plassys MEB 400S or Plassys 500S) by loading onto a sample holder; blowing with an N₂ gun, evaporating 5 nm Ti / 50 nm Au; then lifting off with acetone in a 50°C water bath for ≥ 1 hour and pipette cleaned. Once all metal was lifted off, the sample was transferred to fresh acetone, then isopropyl alcohol, then dried under N₂.

Finally, it was treated again in O₂ plasma (Asher PlasmaFab RF Barrel) at 150 W for 10 minutes.

Thirdly, the nanostructures themselves are to be formed in the wells; this is done by electron beam lithography.

The sample was blown with an N₂ gun then spun coated with PMMA (polymethylmethacrylate) (AR-P 642.04 (200k, 4%), anisole, 100 nm thick) at 4000 RPM for 60 seconds. It was then baked on a hotplate at 180°C for 3 minutes. Next it was blown again with an N₂ gun, and spun coated again this time with PMMA (AR-P 679.02 (950k, 2%), ethyl lactate, 70 nm thick) at 4000 RPM for 60 seconds. It was then baked on a hotplate at 180°C for 3 minutes. Next it was blown again with an N₂ gun, and spun coated again this time with Electra92 (AR-PC 5091 conductive protective coating, Allresist) at 4000 RPM for 60 seconds. It was then baked on a hotplate at 90°C for 2 minutes.

The PMMA was then patterned, in the pattern aligned with that shown in **Fig. 16****,** and according to the desired nanostructure design, using a Raith EBPG5200 electron beam lithography tool. The nanostructure beam parameters were as follows:

| Nanostructure beam parameters | Defined using Beamer (GenlSys GmbH) software | | |
|---|---|---|---|
| | | • 100kV | |
| | | • Write position Grid | |
| | | | • Resolution: 1 nm |
| | | | • Beam Step: 2 nm |
| | | • Mainfield | |
| | | | • Resolution: 1 nm |
| | | | • Size: 1 mm |
| | | • Subfield | |
| | | | • Resolution: 0.5 nm |
| | | | • Size: 4.524 um |
| | | • Fracture Control: | |
| | | | • LRFT (Large Rectangle, Fine Trapezoid) |
| | | | • High Res |
| | | • Field Order: Float | |
| | | • Feature Order: Subfield Compaction | |

| | CJob (Riath) | | |
|---|---|---|---|
| | | • Beam current: 2 nA | |
| | | • Dose: 800 µC/cm² | |

After patterning, the Electra-92 coating was removed by rinsing in reverse osmosis water for 1 minute.

Development in a solution containing methyl isobutyl ketone (MIBK) and isopropyl alcohol (IPA) in a ratio 1:2.5 MIBK:IPA for 1 minute was performed, followed by rinsing in IPA for 1 minute to stop development and N₂ drying.

An O₂ plasma treatment (Asher PlasmaFab RF Barrel) at 80 W for 30 seconds was then performed to remove any resist residue that didn't develop.

The sample was then metallised to form Ti-Au nanostructures (Plassys MEB 400S or Plassys 500S) by loading onto a sample holder; blowing with an N₂ gun, evaporating 5 nm Ti / 50 nm Au; then lifting off with acetone in a 50°C water bath for ≥ 15 minutes and pipette cleaned. Once all metal was lifted off, the sample was transferred to fresh acetone, then isopropyl alcohol, then dried under N₂.

A final O₂ plasma treatment (Asher PlasmaFab RF Barrel) at 150 W for 10 minutes was then performed.

### Nanoarray Design

The nanostructures were patterned as a 750 µm diameter circular pattern of nanostructures (that pattern of nanostructures forming a nanoarray), 4.5 mm separated, to fit into the 700 µm diameter wellplates.

Each nanoarray consisted of squares designed to have a 100 nm x 100 nm shape, with an X- and Y-period of 300 nm between structures.

In alternative embodiments it will be recognised that other nanoarray designs might be used; for example an array consisting of split-rings designed with an outer-radius of 100 nm and inner-radius of 65 nm. A 25 nm gap in the ring is at 0°, 120°, and 240°. The X- and Y-period of the features is suitably 400 nm.

### Rapid Thermal Annealing

A thermal annealing of the thus formed metallised nanostructures was then performed. Using a Jipelec Jetfirst device (thermocouple temperature control) and an N₂ gas environment, a heating/purge protocol as follows was performed:
1. 30 seconds ramp to 75°C;
2. 20 seconds N₂ purge at 75°C;
3. 30 second vacuum at 75°C;
4. 1 second N₂ purge at 75°C;
5. 5 seconds vacuum at 75°C;
6. 10 seconds N₂ purge at 75°C;
7. 40 seconds ramp to 350°C;
8. 30 seconds ramp to 500°C;
9. 600 seconds annealing at 500°C;
10. 10 seconds hold at 0°C;
11. 15 seconds vacuum at 0°C;
12. 15 seconds N₂ purge at 0°C.

### Surface Functionalisation

Using chemistries as discussed above, in particular thiol chemistry, chemical functionalisations were added to each array. It will be recognised that a given array might be left 'blank', that is, without a chemical functionalisation added, in some embodiments.

To make suitable solutions for application to the arrays, 20 µmol (by weight) of the relevant functionalising compound was placed in a glass vial and dissolved in 1 mL ethanol (where ethanol soluble) or water (where not ethanol soluble). 1 mL of ethylene glycol was added to the solution and mixed thoroughly to create a 10mM solution. This procedure was repeated for each relevant functionalising compound to obtain all the necessary solutions of the functionalising compounds.

40 uL of each functionalizing compound solution was loaded into the I-DOT dispensing plate (columnwise). 220 nL of a solution was printed on each blank sensing element as required (for example, where the arrays form a pattern columnwise, with 16 columns, one might use 15 functionalising compounds, one per column, leaving one column under EE only as a control).

The substrate was then removed and placed under a glass cover (petri dish) for 24 hrs [to prevent air currents and rapid evaporation of the ethanol distorting the droplets]. After 24 hrs incubation (at room temperature) the substrate was placed rapidly in a tank of EtOH (volume of at least 500 mL) for c. 10 s. The substrate was then placed in a second clean volume of EtOH and left for 30 s. The substrate was then dried under a stream of nitrogen before 3 further EtOH rinses.

### Solution Preparation - Test Solutions

In order to test the sensitivity of an example device, solutions of test compounds to be sensed were made up. In particular, the compounds trichloroanisole (TCA, also referred to as 'must'), dimethylsulfide (DMS) and iso-alpha acids (also referred to as 'bitter') were made up in solutions which were: 5L Must (TCA 40 ng/L); 7L Must (TCA 29 ng/L); 10L Must (TCA 20 ng/L); 1 L DMS (255 µg/L); 1.3 L DMS (196 µg/L); 2 L DMS (128 µg/L); 1 L Bitter (+5 IBU); 1.5 L Bitter (+c. 3 IBU); and 2 L Bitter (+c. 2 IBU). These compounds represent commonly found 'taints' in beer and hence their accurate sensing is valuable.

The base to which the additives (taints) were added was a standard beer. The taints (TCA, DMS and bitter) were added in the amounts specified.

The 'bitter', TCA and DMS used were purchased from Flavoractiv^{™} (UK). The 'bitter' is described as a mixture of acids from hops and hop oil extracts. It was added to the standard beer in amounts to give a certain additional IBU (International Bitterness Unit) of flavour. This is a well understood term in the add, referring to the perceived bitterness of a solution. Such bitter taste modifiers are provided with a defined IBU 'strength', whereby addition of a known amount of the taste modifier adds a defined level of bitterness on the IBU scale. For example, the bitter used here adds 5 IBU when added to 1 litre of liquid.

### Sensitivity Testing

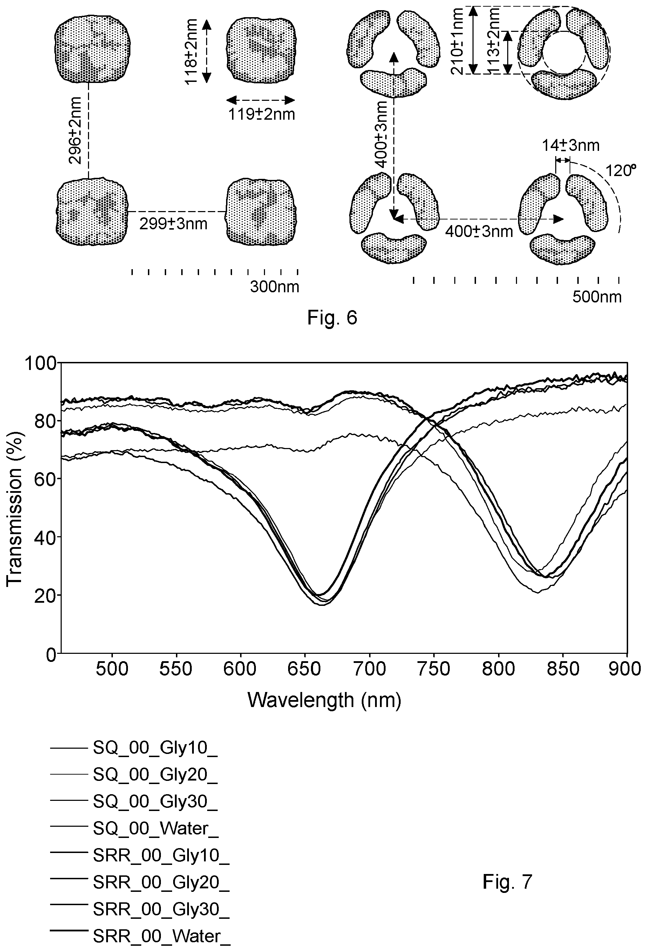

A device having sixteen arrays was produced to test sensitivity against the Test solutions. Following the protocol above, the fifteen of the arrays were functionalised; the sixteenth was left blank (that is, it was not functionalised). The nanostructures were designed to have a substantially square shape, as illustrated in the left hand part of **Fig. 6****,** the squares having a side length of approximately 118.5 nm ± 2.5 nm and a centre-to-centre spacing of approximately 298 nm ± 4 nm.

The functionalising compounds used for the arrays were: 1H,1H,2H,2H-perfluorodecanethiol (PFDT), 1-Dodecanethiol (DDT), 4-mercaptobenzoic acid (MBA), 6-mercapto-1-hexanol (MHOH), 3-amino-5-mercapto-1,2,4-triazole (AMT), 4-nitrothiophenol (NTP), 3,4-dichlorothiophenol (DTP), 1-Octanethiol (OT), 3-mercaptopropionic acid (MPA), 11-mercaptoundecanoic acid (MUA), 2-Phenyletanethiol (PET), 4-mercaptophenylboronic acid (MPBA), 2-furanmethanethiol (FMT), 5,5'-Dithiobis(2-nitrobenzoic acid) (NBA) and L-glutathione (GLU).

The sample for testing (i.e. either the standard beer of one of the Test Solutions of tainted beer described above) was added to a clean disposable petri dish and the (clean and dry) device as a sensor was added to the sample. Each of the 16 functional regions is aligned under a microscope and spectrometer and a spectrum taken as described below in the section "Experimental Setup".

The sensor was then removed from the sample, washed with ethanol and water and dried under a stream of nitrogen. The Test Solution and dish were disposed of and the procedure repeated with the next Test Solution.

The raw spectra were then processed as set out below in the sections "Experimental Setup" and "Data Analysis" to find the minima and the shifts, versus the unadulterated standard beer, were calculated. These shifts are tabulated below as relative sensitivities of the different functionalised arrays to the different taints added in the Test Solutions.

**Table 1 - Sensitivities**

| Sample name | 5 L Must | 7 L Must | 10 L Must | 1 L DMS | 1.3 L DMS | 2 L DMS | 1 L Bitter | 1.5 L Bitter | 2 L Bitter |
|---|---|---|---|---|---|---|---|---|---|
| Technical details: | TCA 40 ng/L | TCA 29 ng/L | TCA 20 ng/L | 255 µg/L | 196 µg/L | 128 µg/L | +5 IBU | +c. 3 IBU | +c. 2 IBU |
| AMT | 0.81 | 1.26 | -0.27 | 1.26 | 0.48 | 1.53 | 0.96 | 1.23 | 1.29 |
| Blank | 1.35 | 1.38 | 1.11 | 2.25 | 1.44 | 2.88 | 2.01 | 1.98 | 1.68 |
| DDT | 2.76 | 5.28 | 4.2 | 5.28 | 4.65 | 4.95 | 5.31 | 5.58 | 5.52 |
| DTP | -6.45 | -7.02 | -6 | -5.64 | -5.43 | -6.03 | -5.22 | -5.37 | -5.49 |
| FMT | 0.69 | 1.59 | 1.92 | 1.83 | 3.93 | 2.55 | 3.99 | 2.88 | 3.39 |
| GLU | 1.5 | 0.18 | 2.04 | 0.12 | 1.74 | 0.33 | 3.06 | 3.39 | 2.52 |
| MBA | 0.33 | 0.33 | 0.84 | 1.05 | 0.93 | 1.5 | 2.91 | 2.97 | 1.32 |
| MHOH | 0.63 | 2.04 | 1.23 | 1.65 | 1.29 | 1.74 | 2.49 | 1.71 | -0.69 |
| MPA | -2.16 | -1.59 | -1.68 | -1.17 | -0.48 | -1.47 | -1.47 | -0.69 | -0.51 |
| MPBA | 0.99 | 0.27 | 0.45 | 1.8 | 1.23 | 1.26 | 1.92 | 2.76 | 2.28 |
| MUA | 1.74 | 0.81 | 2.07 | 1.53 | 1.26 | 1.44 | 1.5 | 1.44 | 1.53 |
| NBA | 0.54 | 0.51 | -0.3 | 0.57 | 2.01 | 0.99 | 1.26 | 0.96 | 1.11 |
| NTP | 0.63 | 0.78 | -0.09 | 0.93 | 2.07 | 0.96 | 2.64 | 2.73 | 1.98 |
| OT | -3.57 | -3.51 | -3.72 | -2.1 | -1.44 | -2.04 | -1.98 | -1.32 | -1.26 |
| PET | 0 | -0.3 | -0.18 | 0.9 | 0.24 | 0.45 | 1.5 | 0.39 | -0.09 |
| PFDT | -1.95 | -1.89 | -1.89 | -0.36 | -0.81 | -1.14 | 0.15 | -0.18 | -0.03 |

A larger number here (= larger shift from the result obtained for the standard, untainted beer) is indicative of a larger effect of the particular Test Solution on the particular array. It can therefore be thought that, by analysis of the various shifts and effects on the different arrays, good discrimination (i.e. good detection of a particular taint) is possible.

For example, if an array shifts a lot for many different taints, that might not be as useful in discrimination as an array which shifts only a small amount, but for only one specific taint. Both responses can be useful, and a combination of them can give high level of discriminatory confidence.

### Experimental Setup

Transmission measurements on the micro-scale were measured on a custom-built microspectrophotometer. Light from a VIS-NIR light source (quartz tungsten halogen light lamp) was used to probe the sensor. A 10x objective was used to couple the transmitted light into an optical fibre attached to a StellarNet Microspectrophotometer (StellarNet Blue Wave, 0.5 nm resolution) with a diffraction grating at the detector for the simultaneous measurement of monochromatic wavelengths of light. This resulted in a spot size of approximately 45 µm. Prior to taking measurements of actual samples, a light reference (a spot from a blank region of the sample) and dark reference (measurement when the shutter was closed) were taken.

Data acquisition settings were as follows:
Integration time: 500 ms
Sample averaging: 3
Episodic data capture of 5 spectra with 3 ms between capture.

Peak position, FWHM, and peak height calculations were performed with a preprocessing of:
0) The data is cut from 450 to 900 nm
1) 3-points average smoothing.

After preprocessing, the minima, FWHM, and peak height are calculated.

### Minima Finding:

For each of the 5 spectra from the episodic data capture, a minima is calculated. For each spectra, the literal mimina of the data is found and the data is cut around this minima by fitting the entire spectra with a high order polynomial (polyfit function). The second derivative of this fit is then used to find the inflection points and the data is then cut at these inflection points. Once this cut has taken place, the remaining peak (trough) is levelled out as to not bias any following steps. The remaining subset of data is 30-point smoothed and the minimum value is taken from this smoothed section. The averaged minima of all 5 spectra in the episodic data-set is output.

### FWHM and Height Finding:

The y-values of first 100 datapoints (after preprocessing) are averaged to give a baseline. The absolute minima (not fit minima) is subtracted from this baseline to find the height of the spectra. The 2 wavelength values closest to half this y-value are then found and the difference between these two wavelength values are the FWHM. The averaged FWHM and averaged height of all 5 spectra in the episodic data-set is output.

### Data Analysis

MATLAB was used to analyze the transmission spectra. The transmission spectrum was smoothed (20 points, meanaverage smoothing) and interpolated (from 0.5 nm to 0.01 nm).

Data can be summarised as full spectra (intensity at all wavelengths) or as four summary metrics (λₘᵢₙ, λ_{half-height L}, λ_{half-height R}, ratio_{LR}) for each nanophotonic region and for each sample. One, two, three or all four of these summary metrics may be found and used in sample analysis. In preferred embodiments, λ_{half-height L}, λ_{half-height R}, and ratio_{LR} (i.e. the ratio between the L and R half-heights) are used.

A table of the relevant data can be prepared, with each row containing data for a particular sample. Each metric that is being used has its own columns, with one such column for each array in the sensor device used in data acquisition. For example, if there are 16 arrays used and the λₘᵢₙ, λ_{half-height L}, λ_{half-height R}, and ratio_{LR} metrics are all being used, there will be 16 x 4 = 64 columns. There will be one column for λₘᵢₙ data from array #1, one column for λ_{half-height L} data from array #1, one column for λₘᵢₙ data from array #2, one column for λ_{half-height L} data from array #2 and so on.

The values used are suitably mean averages of the values obtained over several, for example 2, 3, 4 or 5, repetitions of data acquisition.

Due to the cross-reactive (non-specific nature) of the sensor array it should be noted that input from each and every array element is used in the analysis to identify the sample or analyse the similarity or the difference between two or more samples. Analysis of data collected from a single element alone will not provide information on the sample. In order to combine multiple data from the different array elements, multivariate data analysis methods can be used.

In software such as JMP Pro 16, the LDA function is selected and the 1st column (containing the sample labels) is used as the classifier or categories, and the data from each sensor as the covariates. The software performs a discriminant analysis on the data and reports the likely group membership for each row of data based on its proximity to the calculated centroids of each category. The closer the sample lies to the centroid, the more likely it is to belong to that category.

If a tainted sample lies close to the untainted sample centroid, then this would be a misclassification by the algorithm - a high number of misclassifications would imply that a given sensor cannot determine the difference between either untainted sample and tainted sample; or if two taints are 'confused' by the algorithm, it would imply that the sensor can determine tainted and non-tainted samples, but not what the taint might be.

The number of mislabelled samples (samples that lie closer to the incorrect centroid than their designated label centroid) is calculated, and a percentage classification accuracy can be determined, and a 'confusion matrix' is produced by the software to show which samples are misclassified into which categories.

In some embodiments, LDA is carried out using the sample as the training classifier (e.g. either grouped as sample, or per sample concentration) and either the full spectra or summary metrics as variable. In the case of using full spectra a principal component analysis (PCA) pre-classifier may be applied by the software to linearise the data (JMP Pro 16).

### Results and Discussion

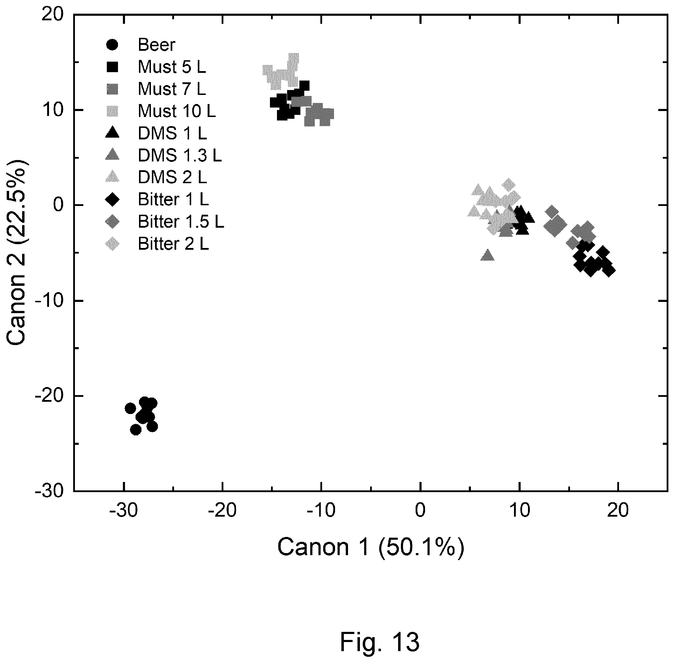

**Figs. 13-15** show data generated from analysis of the samples per the above Table 1. The standard beer against which sensitivities were judged is illustrated by circular marker. The three grades of 'must' tainted test solution are illustrated by square markers; the three grades of 'DMS' tainted test solution by triangular markers; and the three grades of 'bitter' tainted test solution by diamond markers. Each marker represents a single iteration of the testing procedure, which was repeated multiple times for each test solution.

**Fig. 13** plots in two dimensions **Canon 1 and Canon 2,** through which it can be seen that the untainted beer sample is very well distinguished from the tainted beer samples by the exemplified sensor device. The 'must' tainted samples are also well distinguished, and the concentration of taint there quite distinct too. The 'DMS' and 'bitter' tainted samples are reasonable well distinguished, with a little overlap between the most concentrated 'DMS' samples and the least concentrated 'bitter' samples.

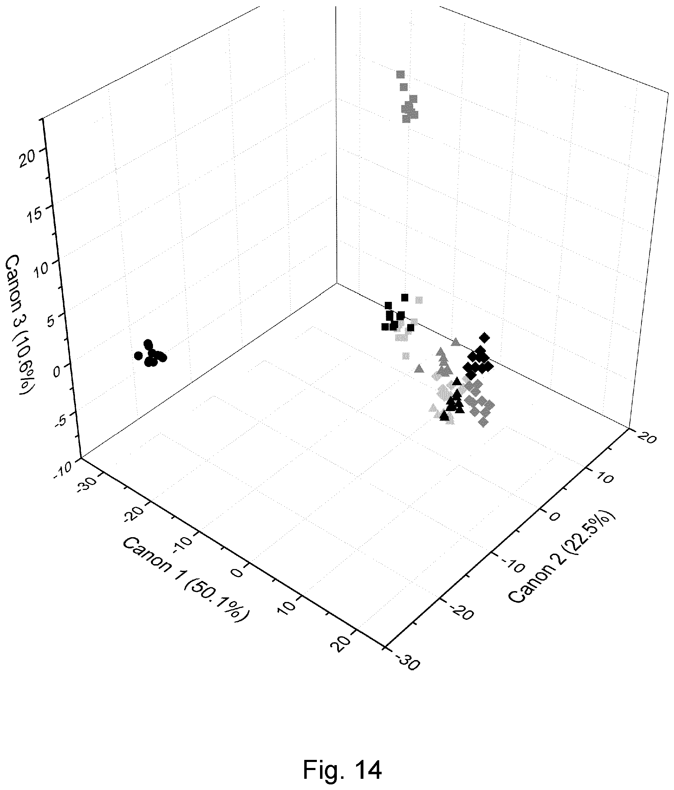

However, **Fig. 14** demonstrates that by adding a third dimension, **Canon 3,** significantly better resolution between those groups is possible.

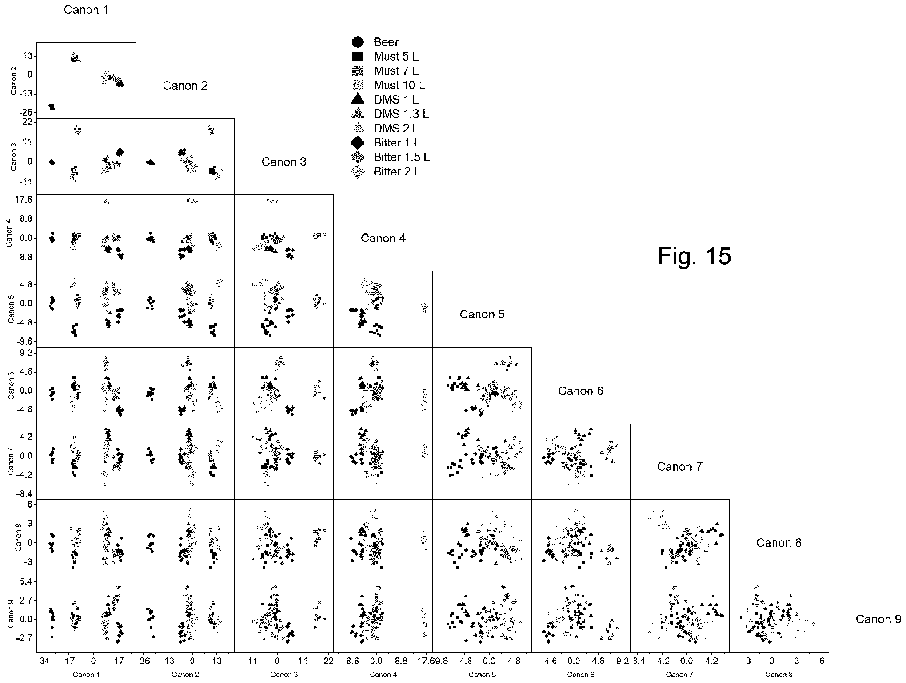

When **Canons 1-9** are all considered, in **Fig. 15****,** the discrimination is very clear.

[It will be recognised by those skilled in the art that these canonical plots are generated by the LDA data analysis method: Canon 1 describes the most separation/discrimination in the model, Canon 2 the second most and so on. Percentages on the axes represent the amount of discrimination described by that linear axis.]

These Figures therefore illustrate the excellent sensing capabilities enabled by the present invention, with clear discernment and identification not only of the presence of beer taints but also identification of what those taints are and even their concentration. Such sensitivity is of huge potential value. While it is demonstrated here for beer, one industry in which the technology might be applied, similar principles can be applied usefully to a variety of different areas to sense the presence, and suitably type and concentration, of an analyte or analytes (e.g. a taint) in a fluid (e.g. a beverage such as beer).

Discussed above are the inventors' developments relating to nanostructure design. In particular, advantages which can be obtained by the use of a 'split ring' design, schematically illustrated in **Fig. 3(B)****,** and a 'square' design, schematically illustrated in **Fig. 3(A)****.** These advantages are illustrated in **Figs. 6, 7****,** **8** **and** **9****.**

**Fig. 6** shows SEM imagery of nanostructures actually deposited according to the schematic designs of **Fig. 3****.** Sensor devices having such nanostructures were fabricated as set out above in the section 'Device Fabrication' and 'Nanoarray Design', of course with changes to the design of the nanostructures as appropriate.

### [Functionalization was not carried out in the test.]

These devices were tested, using the protocol set out in the section 'Experimental Setup', against a series of test solutions. The spectra obtained are illustrated in **Fig. 7****.**

The test solutions used comprised water with certain concentrations of glycerol added. The spectra are coded accordingly:
SQ = square nanostructure design
SRR = split ring nanostructure design
Gly10 = 10% glycerol (by volume) in water used as test solution
Gly20 = 20% glycerol (by volume) in water used as test solution
Gly30 = 30% glycerol (by volume) in water used as test solution
Water = water used as test solution.

From **Fig. 7** it can clearly be seen that the 'SQ' series of spectra have minima lying around the 670 nm wavelength, but more importantly are relatively closely packed at that minimum and indeed generally throughout the spectra. On the other hand, the 'SRR' series of spectra have minima around the 830 nm wavelength but are much more distinct, not only there but throughout the spectra.

This clearer separation of the spectra demonstrates that the split ring design is much better able to sense an analyte (here, glycerol) in a fluid (here, water) than the square design. The data generated in its spectra can be analysed to give much better discrimination.

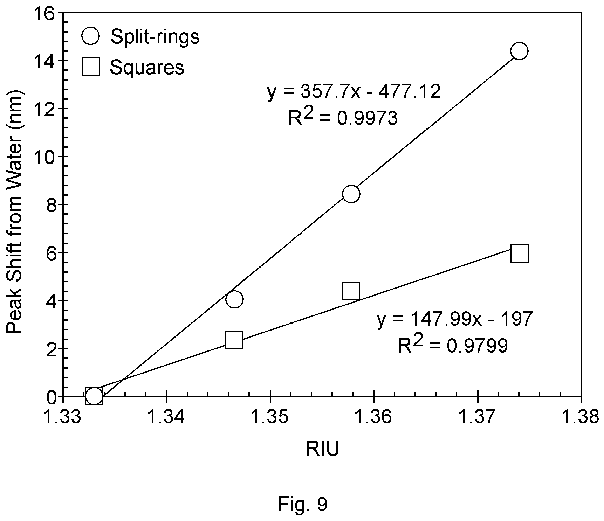

The improved sensing provided by a split ring design is further illustrated in **Fig. 9****.**

To generate these data, refractive index measurements were taken using a Kern ABBE Refractometer (Model 0RT1RS). For the glycerol solutions, the results were:

| | Measured RI |
|---|---|
| Water | 1.333 |
| 10% glycerol (Gly10) | 1.3465 |
| 20% glycerol (Gly20) | 1.3578 |
| 30% glycerol (Gly30) | 1.3739 |

By looking at the peak shift from water (nm) for each glycerol solution in **Fig. 7****,** the points of the graph of **Fig. 9** can be generated (circles for the split ring nanostructure design, squares for the square nanostructure design). Fitting a line to the points gives the effective formulae shown in **Fig. 9****.**

It is well understood that (relative) sensitivity is related to the slope of the fitted line in such analysis. The slope of the split ring line (357.7) is more than 2.4 times that of the slope of the square line (147.99). Accordingly the split ring nanostructure design can be said to be 2.4 times more sensitive than the square nanostructure design.

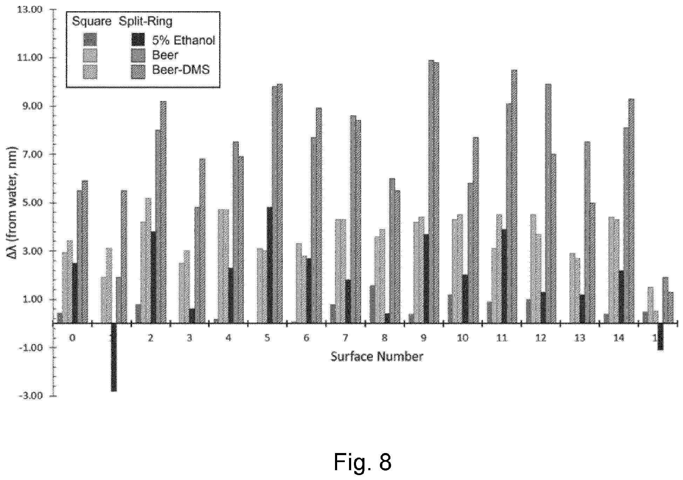

**Fig. 8** shows the results of another investigation into the improvement provided by the split ring nanostructure design as compared to the square one. Devices were fabricated as set out above, in the section 'Device Fabrication' and 'Nanoarray Design', of course with changes to the design of the nanostructures as appropriate.

Functionalization was carried out according to the 'surface functionalization' section above. 16 arrays present were functionalised as follows:

| Array (Surface) Number | Functionalising compound | Abbreviation | Solvent |
|---|---|---|---|
| 0 | None | N/A | N/A |
| 1 | 1-octanethiol | OT | EtOH |
| 2 | 1-dodecanethiol | DDT | EtOH |
| 3 | 2-phenylethanethiol | PET | EtOH |
| 4 | 4-mercaptobenzoic acid | MBA | EtOH |
| 5 | 11-mercaptoundecanoic acid | MUA | EtOH |
| 6 | 6-mercapto-1-hexanol | MHOH | EtOH |
| 7 | 2-furanmethanethiol | FMT | EtOH |
| 8 | 3-amino-5-mercapto-1,2,4-triazole | AMT | EtOH |
| 9 | L-glutathione | GLU | Water |
| 10 | 4-mercaptophenylboronic acid | MPBA | EtOH |
| 11 | 1H,1H,2H,2H-perfluorodecanethiol | PFDT | EtOH |
| 12 | 3-mercaptopropionic acid | MPA | EtOH |
| 13 | 5,5'-dithiobis(2-nitrobenzoic acid) | NBA | EtOH |
| 14 | 4-nitrothiophenol | NTP | Water |
| 15 | 3,4-dichlorothiophenol | DTP | Water |

Spectra were obtained following the 'Experimental Setup' section above, suing three test solutions: a solution of 5% ethanol in water; a standard beer; and the standard beer with 255 µg/L of dimethyl sulfide added. A comparison spectrum for water was also obtained.

The change in resonance peak as compared to water for each array was quantified. The changes are plotted in **Fig. 8****:** for each surface (=array), the bars left to right represent the changes for 5% ethanol (square design); beer (square design); beer+DMS (square design); 5% ethanol (split ring design); beer (split ring design); and beer+DMS (split ring design).

It is apparent that significantly larger shifts are observed for the split ring design sensors, universally across all the illustrated functionalisations. Therefore it is clear that the split ring design sensor devices are significantly better at distinguishing the test mixtures (5% ethanol, beer, and tainted beer) from water.

Also discussed above are the inventors' insights into improvements that are available by subjecting the deposited nanostructures to a thermal annealing procedure. These improvements make it easier to track small peak shifts in the sensor.

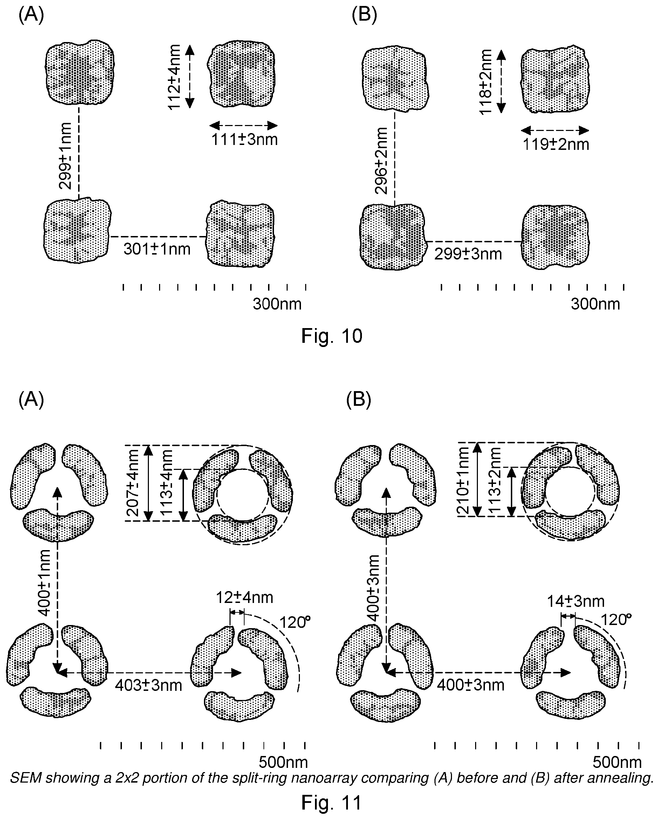

**Figs. 10 and 11** show the effect of the annealing procedure set out above (section 'Rapid Thermal Annealing') on both square **(****Fig. 10****)** and split ring **(****Fig. 11****)** nanostructure designs as originally deposited according to the design shown in **Fig. 3****.** It can be seen that the annealed structures (B) are slightly enlarged as compared to the same structures before annealing (A).

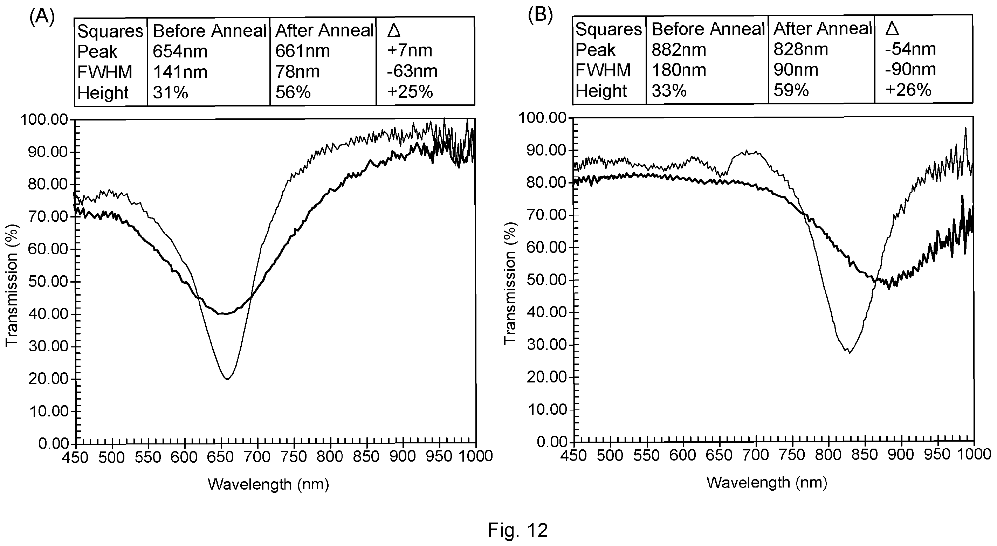

Comparison transmission spectra are shown in **Fig. 12** for (A) square nanostructures and (B) split ring nanostructures (fabricated as above, using water as the test solution). It can be seen that the spectra after annealing are, in both cases, deeper and sharper because of the improved optical resonance. This improvement makes it easier to monitor small shifts in the resonance wavelength.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined by the appended claim.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means +/- 10% unless otherwise stated.

### References

A number of publications are cited above in order to more fully describe and disclose the invention and the state of the art to which the invention pertains. Full citations for these references are provided below.
A1. Jennings W, Shibamoto T. Qualitative Analysis of Flavor and Fragrance Volatiles by Glass Capillary Gas Chromatography. 1 ed: Elsevier; 2012.
A2. Ng SC, Ong TT, Fu P, Ching CB. Enantiomer separation of flavour and fragrance compounds by liquid chromatography using novel urea-covalent bonded methylated beta-cyclodextrins on silica. Journal of Chromatography A. 2002;968(1-2):31-40.
A3. Rodriguez-Mendez ML, De Saja JA, Gonzalez-Anton R, GarciaHernandez C, Medina-Plaza C, Garcia-Cabezon C, et al. Electronic Noses and Tongues in Wine Industry. Frontiers in Bioengineering and Biotechnology. 2016;4.
A4. Kermani BG, Schiffman SS, Nagle HT. Performance of the Levenberg-Marquardt neural network training method in electronic nose applications. Sens Actuators, B. 2005;110(1):13-22.
A5. D'Amico A, Pennazza G, Santonico M, Martinelli E, Roscioni C, Galluccio G, et al. An investigation on electronic nose diagnosis of lung cancer. Lung Cancer. 2010;68(2):170-6.
A6. Han J, Ma C, Wang B, Bender M, Bojanowski M, Hergert M, et al. A Hypothesis-Free Sensor Array Discriminates Whiskies for Brand, Age, and Taste. Chem. 2017;2(6):817-24.
A7. Mimendia A, Gutierrez JM, Leija L, Hernandez PR, Favari L, Munoz R, et al. A review of the use of the potentiometric electronic tongue in the monitoring of environmental systems. Environmental Modelling & Software. 2010;25(9):1023-30.
A8. Askim JR, Mahmoudi M, Suslick KS. Optical sensor arrays for chemical sensing: the optoelectronic nose. Chem Soc Rev. 2013;42(22):8649-82.
A9. Peveler WJ, Yazdani M, Rotello VM. Selectivity and Specificity: Pros and Cons in Sensing. Acs Sensors. 2016;1(11):1282-5.
A10. Persaud K, Dodd G. ANALYSIS OF DISCRIMINATION MECHANISMS IN THE MAMMALIAN OLFACTORY SYSTEM USING A MODEL NOSE. Nature. 1982;299(5881):352-5.
A11. Lu C, Lipson RH. Interference Lithography: A Powerful Tool for Fabricating Periodic Structures. Laser & Photonics Reviews. 2010;4(4):568-80.
A12. WO 2021/156346 A1
A13. Gerard Macias, Justin R. Sperling, William J. Peveler, Glenn A. Burley, Steven L. Neale and Alasdair W. Clark, Whisky tasting using a bimetallic nanoplasmonic tongue, Nanoscale, 2019, 11, 15216-15223, DOI: 10.1039/C9NR04583J
A14. US 2011/0164252 A1

## Claims

1. A localised surface plasmonic sensing device (10) comprising:
a substrate (12);
a first array (20) of localised surface plasmon resonance island structures (14) on the substrate;
a second array (22) of localised surface plasmon resonance island structures (14) on the substrate:
a third array of localised surface plasmon resonance island structures on the substrate;
a fourth array of localised surface plasmon resonance island structures on the substrate, wherein:
the first, second, third and fourth arrays are located to be spaced apart and isolated from each other on the substrate;
the localised surface plasmon resonance island structures of the first, second, third and fourth array respectively have first, second, third and fourth surface functionalisations for selective interaction with respective analytes;
the first, second, third and fourth surface functionalisations are different to each other and, other than the different surface functionalisation, the localised surface plasmon resonance island structures of the first, second, third and fourth array have the same composition as each other;
and wherein the surface functionalisations on the substrate include at least:
a surface functionalisation derived from 1-Dodecanethiol,
a surface functionalisation derived from 6-mercapto-1-hexanol,
a surface functionalisation derived from 3-amino-5-mercapto-1,2,4-triazole, and
a surface functionalisation derived from 3,4-dichlorothiophenol.

## Patentansprüche

1. Lokalisiertes-Oberflächenplasmon-Abtastvorrichtung (10), die Folgendes umfasst:
ein Substrat (12),
eine erste Anordnung (20) von lokalisierten Oberflächenplasmonresonanz-Inselstrukturen (14) auf dem Substrat,
eine zweite Anordnung (22) von lokalisierten Oberflächenplasmonresonanz-Inselstrukturen (14) auf dem Substrat,
eine dritte Anordnung von lokalisierten Oberflächenplasmonresonanz-Inselstrukturen auf dem Substrat,
eine vierte Anordnung von lokalisierten Oberflächenplasmonresonanz-Inselstrukturen auf dem Substrat;
wobei:
die erste, zweite, dritte und vierte Anordnung so angeordnet sind, dass sie auf dem Substrat voneinander beabstandet und isoliert sind;
wobei die lokalisierten Oberflächenplasmonresonanz-Inselstrukturen der ersten, zweiten, dritten und vierten Anordnung jeweils eine erste, zweite, dritte und vierte Oberflächen-Funktionalisierung zur selektiven Wechselwirkung mit entsprechenden Analyten aufweisen;
wobei sich die erste, zweite, dritte und vierte Funktionalisierung voneinander unterscheiden und wobei die lokalisierten Oberflächenplasmonresonanz-Inselstrukturen der ersten, zweiten, dritten und vierten Anordnung abgesehen von der unterschiedlichen Oberflächen-Funktionalisierung dieselbe Zusammensetzung wie die anderen aufweisen; und
wobei die Oberflächen-Funktionalisierungen auf dem Substrat zumindest eine der folgenden umfassen:
eine von 1-Dodecanthiol abgeleitete Oberflächen-Funktionalisierung,
eine von 6-Mercapto-1-hexanol abgeleitete Oberflächen-Funktionalisierung,
eine von 3-Amino-5-mercapto-1,2,4-triazol abgeleitete Oberflächen-Funktionalisierung und
eine von 3,4-Dichlorthiophenol abgeleitete Oberflächen-Funktionalisierung.

## Revendications

1. Dispositif de détection plasmonique de surface localisée (10), comprenant :
un substrat (12) ;
un premier réseau (20) de structures d'îlot de résonance plasmonique de surface localisée (14) sur le substrat ;
un deuxième réseau (22) de structures d'îlot de résonance plasmonique de surface localisée (14) sur le substrat ;
un troisième réseau de structures d'îlot de résonance plasmonique de surface localisée sur le substrat ;
un quatrième réseau de structures d'îlot de résonance plasmonique de surface localisée sur le substrat, dans lequel :
les premier, deuxième, troisième et quatrième réseaux sont situés pour être espacés et isolés les uns des autres sur le substrat ;
les structures d'îlot de résonance plasmonique de surface localisées des premier, deuxième, troisième et quatrième réseaux présentent respectivement des première, deuxième, troisième et quatrième fonctionnalisations de surface pour une interaction sélective avec des analytes respectifs ;
les première, deuxième, troisième et quatrième fonctionnalisations de surface sont différentes les unes des autres et, outre la fonctionnalisation de surface différente, les structures d'îlot de résonance plasmonique de surface localisées des premier, deuxième, troisième et quatrième réseaux présentent la même composition les unes par rapport aux autres ;
et dans lequel les fonctionnalisations de surface sur le substrat incluent au moins :
une fonctionnalisation de surface dérivée de 1-dodécanethiol,
une fonctionnalisation de surface dérivée de 6-mercapto-1-hexanol,
une fonctionnalisation de surface dérivée de 3-amino-5-mercapto-1,2,4-triazole, et
une fonctionnalisation de surface dérivée de 3,4-dichlorothiophénol.
